# EUROPEAN PATENT APPLICATION

(11) **EP 2 366 796 A1**
(43) Date of publication of application: **21.09.2011**
(21) Application number: 10002443.9
(22) Date of filing: 09.03.2010
(51) Int. Cl.: C12Q 1/68, G01N 33/50

(54) **Means and methods for identifying compounds altering nuclear localization of proteins**

(71) Applicant: Friedrich-Alexander-Universität Erlangen-Nürnberg, 91054 Erlangen (DE)
(72) Inventor: Hillen, Wolfgang, 91056 Erlangen (DE); Stöckle, Christoph, 91058 Erlangen (DE); Goeke, Dagmar, 91080 Spardorf (DE)
(74) Representative: Wachenfeld, Joachim

(57) **Abstract**

This invention relates to a eukaryotic cell comprising: (a) a first nucleic acid comprising a reporter gene under the control of a response element; (b) a second nucleic acid comprising a gene encoding a transcription factor, said transcription factor being capable of binding to said response element, thereby controlling expression of said reporter gene; and (c) a third nucleic acid encoding a fusion protein, said fusion protein comprising: (i) a polypeptide of interest; and (ii) a peptide being capable of binding to said transcription factor, thereby controlling the activity of said transcription factor.

## Description

This invention relates to a eukaryotic cell comprising: (a) a first nucleic acid comprising a reporter gene under the control of a response element; (b) a second nucleic acid comprising a gene encoding a transcription factor, said transcription factor being capable of binding to said response element, thereby controlling expression of said reporter gene; and (c) a third nucleic acid encoding a fusion protein, said fusion protein comprising: (i) a polypeptide of interest; and (ii) a peptide being capable of binding to said transcription factor, thereby controlling the activity of said transcription factor.

In this specification, a number of documents including patent applications and manufacturers' manuals are cited. The disclosure of these documents, while not considered relevant for the patentability of the invention, is herewith incorporated by reference in its entirety. More specifically, all reference documents are incorporated by reference to the same extent as if each individual document was specifically and individually indicated to be incorporated by reference.

The nuclear membrane divides the eukaryotic cell into compartments between which a regulated transport of proteins and nucleic acids occurs through nuclear pore complexes (NPC). Regulation of nuclear transport takes place at different hierarchical levels including the NPC, transport receptors like the importin or exportin protein families and the cargo itself (Terry et al., 2007). This sophisticated process of shuttling proteins between the cytoplasm and the nucleus is of vital importance for a variety of biological processes (Terry et al., 2007). Especially proteins participating in replication, transcription and its regulation, including transcription factors, and the splicing machineries must be transported into the nucleus to exert their proper function. Transcription factor transport into the nucleus is frequently dependent on modifications introduced by upstream signaling chains. Changes in the expression levels of proteins involved in nuclear transport, like the exportin Crm1 and the importins Karyopherin β1 (Kpnβ1) and Karyopherin α2 (Kpnα2) (van der Watt et al., 2009) have been detected in a variety of different types of cancer cells (Kau, 2004). There have been attempts to develop the Crm1 inhibitor Leptomycin B for cancer therapy, but this compound is not suitable as an anticancer drug due to its high toxicity (Newlands et al., 1996). Furthermore, specific karyopherins/importins are either down- or up-regulated in several cancer types (Kau et al., 2004, Winnepenninckx et al., 2006, Dahl et al., 2006). Since these proteins are involved in facilitating the nuclear transport of transcription factors, changes in their expression levels lead to a lack of proper control of gene expression in these cancer cells. Another prominent example involves the "guardian of the genome" p53 (Lane, 1992) which is malfunctioning in over 50% of all human cancers. This is often the result of an uncontrolled nucleo-cytoplasmic shuttling brought about by mutations in proteins which control stability or localization of p53 leading to decreased levels of the tumor suppressor protein p53 inside the nucleus. This contributes to tumorgenesis since p53-driven elimination of aberrant cells via apoptosis or cell cycle arrest represents a crucial component of the host defense against tumor development. A further prominent example is illustrated by β-catenin responding to the extra-cellular matrix protein osteopontin (OPN) which is known to be overexpressed in a variety of malignancies including prostate, lung, breast, colorectal and stomach cancer (Luu *et al.,* 2004) (Robertson & Chellaiah, 2010). OPN triggers a signaling cascade leading to phosphorylation of β-catenin via ILK, P13K and AKT kinase which causes stabilization and nuclear import of β-catenin. By interacting with the T cell factor (TCF) family of transcription factors, β-catenin leads to the up-regulation of several genes important for cancer progression, e.g., the proto-oncogene c-Myc (Morin, 1999). These few examples may serve to represent the large number of proteins for which imbalanced nuclear localization contributes to cancer development and rapid growth of malignant cells. All of these proteins, more examples of which are shown in Table 1 below, are targets for the novel screening method as disclosed herein, which can be executed conveniently in a high throughput screening format.

**Table 1: Proteins implicated in cancer**

| | | |
|---|---|---|
| Examples of proteins are listed that either interfere with nucleo-cytoplasmic signaling pathways, or that exhibit altered nuclear localization. | | |

| ***Protein*/*protein family*** | ***Function*** | ***Reference*** |
|---|---|---|
| β-Catenin | transcription factor (Wnt signaling) | (Morin, 1999) |
| Crm1 | exportin | (Kau et al., 2004), (van der Watt et al., 2009) |
| Kpnβ1 | importin | (Kau et al., 2004), (van der Watt et al., 2009) |
| MDM2 | p53-interacting protein/E3 ligase | (Momand et al., 2000) |
| PARC | p53-associated cytoplasmic protein | (Nikolaev and Gu, 2003) |
| Nucleophosmin | nuclear chaperone | (Falini et al., 2009; Grisendi et al., 2006) |
| Smad | signaling proteins (TGF-β - Smad signaling) | (Halder et al., 2008; Kloth et al., 2008; Lu et al., 2007) |

Nuclear transport is also essential for most viruses because their nucleic acids are either replicated in the nucleus of the infected cell or they interfere with host signaling chains, immune responses and nuclear transport processes to complete their life cycles. Among viral infections, Influenza virus is a typical example due to its high rate of antigenic change giving rise to new, potentially highly virulent strains by combining the haemagglutinin subtypes (H1-H16) with all neuramidase subtypes (N1-N9). The relevance of influenza A infections as a public health threat is nowadays obvious with outbreaks of the avian virus H5N1 during the last decade and currently of the swine-originated virus H1N1. The nucleo-cytoplasmic transport processes crucial for Influenza virus A infection have been reviewed (Boulo et al., 2007). Virus particles are internalized by receptor-mediated endocytosis, which subsequently leads to acidification of the endosome. After membrane fusion, viral ribonucleoprotein complexes (vRNPs) gain access to the cytoplasm of the infected cell. vRNPs consist of single-stranded, minus-sense RNA covered by several copies of the nucleoprotein NP and a single copy of an RNA-dependent RNA polymerase composed of the subunits PA, PB1 and PB2. All of these proteins contain their own nuclear localization signal (NLS); however, it is unclear which of these NLS signals is important for the nuclear import process. Several other viral proteins must undergo nuclear import or export during viral infection as well; see Table 2 below.

**Table 2: Proteins of influenza virus A trafficking between the cytoplasm and the nucleus**

| ***Protein*** | ***Function*** | ***Reference*** |
|---|---|---|
| RNA-dependent RNA-polymerase (subunits PA, PB1, PB2) | transcription of viral RNA | (Kemler et al., 1994) |
| M1 | association with vRNPs; role in export of vRNPs | (Zhirnov, 1992) |
| NEP | role in export of vRNPs | (Neumann et al., 2000) |
| NP | nucleoprotein covering viral RNA | (Wang et al., 1997) |
| NS1 | inhibition of antiviral host defenses | (Min and Krug, 2006) |

| | | |
|---|---|---|
| vRNP, viral ribonucleoprotein | | |

There are several molecular strategies to treat viral infections (reviewed in Leyssen et al., 2008) and presently about 40 antiviral drugs are licensed for use in humans. However, only a limited number of drugs are available for the treatment of highly pathogenic RNA viruses like Influenza A. These include the M2 channel inhibitors amantadine and rimantadine, and the neuraminidase inhibitors zanamivir and oseltamivir, the latter one known as Tamiflu™. Due to mutations within the neuraminidase gene, Tamiflu™ resistance has been reported (de Jong et al., 2005), (Le et al., 2005). Considering that nucleo-cytoplasmic transport processes are of vital importance for viral replication, interference with such processes constitutes an attractive novel therapeutic approach in Influenza virus chemotherapy. Molecules inhibiting either nuclear import or export of an important viral protein would address a novel target distinct from M2 or neuraminidase and might open up novel therapies. The means and methods disclosed herein can be used to identify novel lead structures for therapeutic interference with nuclear transport of these potential targets, and hence, lead to novel anti-Influenza drugs. This potential exists also for other highly pathogenic viruses as listed in Table 3. The Ebola virus protein VP24 and the SARS virus protein ORF6 interfere with the host immune response by disrupting the nuclear import of phosphorylated STAT1 which ultimately leads to a decrease in transcription of STAT1-dependent gene expression necessary for an adequate immune response (Frieman et al., 2007) (Reid et al., 2006). In general, the method described here can be applied to any virus according to the Baltimore classification system (Baltimore, 1971) when either viral replication requires nucleo-cytoplasmic shuttling or viral components either disrupt host signaling chains or other host processes involving nucleo-cytoplasmic transport necessary to combat viral infection. The Baltimore classification groups viruses into families depending on their type of genome and their replication mechanism. Viruses are classified into double-stranded DNA viruses (including the Order *Herpesvirales),* single-stranded DNA viruses (including the Family *Parvoviridae),* double-stranded RNA viruses (including the Family *Reoviridae),* positive sense single-stranded RNA viruses (including the Family *Picornaviridae),* negative sense single-stranded RNA viruses (including the Family *Filoviridae),* positive sense single-stranded RNA viruses that replicate through a DNA intermediate (including the Family *Retroviridae)* and double-stranded DNA viruses that replicate though a single-stranded RNA intermediate (including the Family *Hepnaviridae).* Examples of pathogenic members from these families are given in Table 3, but are not limited thereto.

**Table 3: Families of highly pathogenic viruses**

| ***Viral family*** | ***Disease*** | ***Reference*** |
|---|---|---|
| Arenaviridae | Lassa fever | (Geisbert and Jahrling, 2004) |
| Bunyaviridae | Congo hemorrhagic fever | (Geisbert and Jahrling, 2004), (Whitehouse, 2004) |
| Coronaviridae | SARS (Severe Acute Respiratory Syndrome) | (Cheng et al., 2007), (Frieman, 2007) |
| Filoviridae | Ebola & Marburg hemorrhagic fever | (Balter, 2000; Geisbert and Jahrling, 2004), (Reid, 2006) |
| Flaviviridae | Yellow fever | (Ellis and Barrett, 2008; Geisbert and Jahrling, 2004) |
| Herpesviridae | Burkitt's lymphoma, Kaposi's sarcoma | (Crawford, 2001), (Chang, 1994) |
| Retroviridae | AIDS, adult leukemia | Suzuki & Craigie, 2007) |
| Orthomyxoviridae | Influenza | (Khanna et al., 2008) |

In general, nuclear transport of proteins is essential for DNA viruses as well, and hence this strategy applies to the development of antiviral drugs as therapeutics for all viral diseases.

Early analysis of nuclear transport was done by electron microscopy (EM) to detect the location of gold-conjugated proteins microinjected into the cytoplasm of amoebas (Feldherr, 1962; Feldherr and Marshall, 1962) or amphibian oocytes (Feldherr et al., 1984). This method was more generally applicable when gold-conjugated antibodies became available to immunologically detect the subcellular localization of distinct proteins (Radu et al., 1995; Rout et al., 2000). Furthermore, the combination of digitonin-permeabilized cells, gold-conjugated nuclear proteins and EM allowed to study the nuclear import of the human transcription factor PU.1 in HeLa cells (Zhong et al., 2006). Another method to analyze nuclear transport is based on the preparation of cytoplasmic and nuclear extracts from cell lines. This method relies on a hypotonic lysis buffer allowing the separation of intact nuclei from the cytoplasm (Dyer and Herzog, 1995). The nuclear extract preparation is then followed by immunological detection of nuclear proteins via Western blot analysis. Clearly, none of these methods is amenable to high throughput screening (HTS).

Nowadays, fluorescent proteins like GFP and derivatives thereof are used to tag endogenous proteins within living cells and observe their subcellular location by fluorescence microscopy. Due to the size of these fluorescent protein tags however, not all proteins tolerate such fusions without impairing their normal function. It has been shown that fusions to GFP can lead to mislocalization of the analyzed protein within the cell (Lisenbee et al., 2003). Furthermore, GFP fusions to certain viral proteins can compromise their function leading to non-infectious viruses (Thomas and Maule, 2000). Hence, it would be advantageous to have alternative strategies to specifically label nuclear proteins of interest without compromising their functionality. This is currently accomplished by labeling proteins with short affinity tags which are recognized by antibodies. Furthermore, a variety of chemical methods exist which allow labeling of proteins with small fluorescent dyes. This offers a further possibility to analyze protein localization, but lacks the precision of genetically encoded tags. Visualization of the target protein may hence be accomplished by a variety of methods, but all of these protocols require microscopic analysis of the location of the target protein in the cell and, hence, none of them is suited for HTS.

The technical problem underlying the present invention can therefore be seen in the provision of alternative or improved means and methods for identifying compounds which are capable of interfering with the nuclear localization of a protein.

Accordingly, this invention relates to a eukaryotic cell comprising: (a) a first nucleic acid comprising a reporter gene under the control of a response element; (b) a second nucleic acid comprising a gene encoding a transcription factor, said transcription factor being capable of binding to said response element, thereby controlling expression of said reporter gene; and (c) a third nucleic acid encoding a fusion protein, said fusion protein comprising: (i) a polypeptide of interest; and (ii) a peptide being capable of binding to said transcription factor, thereby controlling the activity of said transcription factor.

The term "eukaryotic cell" as used herein has the meaning as established in the art. In particular, a eukaryotic cell comprises a nucleus which is confined by a nuclear membrane.

The eukaryotic cell according to the invention will generate a detectable signal originating from the expressed reporter gene provided by the first nucleic acid. Expression of the reporter gene is controlled by the transcription factor encoded by the second nucleic acid. The activity of the transcription factor, and hence the expression of the reporter gene is regulated by the fusion protein provided by the third nucleic acid. Therefore, the expression level of the reporter gene is indicative of the subcellular localization of the fusion protein. Given its design, the eukaryotic cell according to the invention provides an easily measurable readout amenable to HTS and is therefore suitable for applications which are further detailed below and include a method of determining the presence of a polypeptide of interest in the nucleus as well as a method of identifying a compound capable of altering the nuclear localization of a polypeptide of interest.

It is understood that the eukaryotic cell according to the invention is not part of the human organism and preferably not of the organism of an animal. In other words, it is preferred that said eukaryotic cell is present *in vitro* or ex *vivo,* such as an isolated cell or a cell in culture.

The eukaryotic cell according to the main embodiment comprises the three nucleic acids according to features (a), (b) and (c). It is understood that the main embodiment may also be realized such that the features of two or of all three nucleic acids are provided by a single nucleic acid. For example, the eukaryotic cell may comprise a nucleic acid comprising a reporter gene under the control of a response element and furthermore a gene encoding a transcription factor, said transcription factor being capable of binding to said response element. Such a nucleic acid is an implementation of features (a) and (b) of the main embodiment. Generally speaking, features (a), (b) and (c) may be realized by less than three, i.e., one or two distinct nucleic acids.

The first nucleic acid comprises a reporter gene under the control of a response element. In other words, and depending on the status of the response element, expression of the reporter gene either occurs or does not occur, or occurs at varying levels. This implies that the first nucleic acid also comprises a suitable promoter. Exemplary or preferred promoters are described in the examples enclosed herewith and may be used in conjunction with any reporter gene and any response element, provided that the requirements of the main embodiment are met. Further useful or preferred promoters are as follows. Minimal promoters hooked up to Tet response elements are typically derived from, but not limited to the CMV immediate early promoter/enhancer (Gossen & Bujard, 1992; Agha-Mohammadi et al., 2004), the thymidine kinase promoter (Gossen & Bujard, 1992), the murine mammary tumor virus promoter (Hofmann et al., 1997) and the serum response factor α promoter (Go & Ho, 2002). Tet response elements can also be used to control native promoters, like the murine phospho glycerate kinase promoter (Szulc et al., 2006) or combined promoters derived from CMV or SV40 virus promoter elements (Krueger et al. 2006).

The above-mentioned "status of the response element" refers to whether the transcription factor according to (b) is bound to the response element or not. The response element is typically a region which is upstream of the region encoding the reporter gene and serves as a binding site for the transcription factor. The term "response element" has its meaning as established in the art relating to the control of gene expression. Preferred response elements are described further below. When the cognate transcription factor is bound to the response element, the expression of the reporter gene is increased or decreased as compared to the absence of the transcription factor.

The term "reporter gene" has the meaning as established in the art and refers to a gene which, when expressed, i.e., transcribed and translated, gives directly or indirectly rise to a detectable signal. Preferred reporter genes are described further below.

The second nucleic acid according to (b) encodes the transcription factor which, as described above, controls the expression of the reporter gene. It is understood that the second nucleic acid is equipped with those regulatory elements, in particular a promoter, which permit expression of the second nucleic acid in the eukaryotic cell. These promoters can either be ubiquitously active, like the viral CMV immediate early promoter/enhancer (Gossen & Bujard., 1992) and the EF-1α promoter (Gopalkrishnan et al., 1999) or tissue-specific promoters.
In preferred embodiments, said reporter gene and/or said transcription factor are not naturally occurring in eukaryotic cells.

Also, the third nucleic acid is equipped with those regulatory elements, in particular a promoter, which permit expression of the encoded fusion protein in the eukaryotic cell. In the encoded fusion protein, two components, i.e., a polypeptide of interest and a peptide according to feature (c)(ii) occur together within a single amino acid sequence, wherein such co-occurrence is not observed in nature. As will be apparent from the disclosure of further embodiments of the present invention below, the polypeptide of interest is preferably a polypeptide which is located in the nucleus of the eukaryotic cell, at least partially. In other words, the polypeptide may always be located in the nucleus, or may shuttle between nucleus and cytoplasm or between nucleus and other organelles. Presence in the nucleus may depend on external signals or on the stage of the cell cycle. Examples of such polypeptides as well as of conditions for their presence in the nucleus are described in the background section which is herewith incorporated as part of the disclosure of the invention. Preferably, said polypeptide comprises one or more nuclear localization signals (NLS) within its amino acid sequence. Nuclear localization signals are known in the art. They trigger translocation into the nucleus of a polypeptide comprising one or more such signals within its amino acid sequence. Typically, nuclear localization signals consist of one or more short amino acid sequences which are rich in Arg and/or Lys.

The recited activity of said transcription factor, said activity being controlled by the fusion protein comprising the peptide according to the invention, is preferably its DNA-binding activity, more specifically the capability to bind a response element. Binding of the fusion protein to the transcription factor may either enhance or decrease the capability of said transcription factor to bind said response element.

It is understood that an in-frame fusion is envisaged such that (i) the capability of the peptide to bind and control the transcription factor is retained and (ii) the capability of the polypeptide of interest to be located in the nucleus is retained.

In preferred embodiments, only one type of first nucleic acid, one type of second nucleic acid and/or one type of third nucleic acid are present in the eukaryotic cell. Alternatively, two or more types of any one of the three nucleic acids may be present. For example, nucleic acids encoding two reporter genes and/or nucleic acids encoding two transcription factors and/or nucleic acids encoding two fusion proteins may be present.

The eukaryotic cell may be obtained by transfecting, nucleofecting or transducing an apparent eukaryotic cell with the nucleic acids according to (a), (b) and (c). Means and methods of transfecting, nucleofecting and transducing eukaryotic cells are established in the art and at the skilled person's disposal.

Transfection, nucleofection or transduction may be stable or transient. Any parent eukaryotic cell may be used and transfected, nucleofected or transduced in order to obtain the eukaryotic cell according to the main embodiment. For example, if the parent eukaryotic cell is a HeLa cell, also the resulting eukaryotic cell according to the main embodiment would be referred to as "HeLa cell"; noting that key properties of the parent HeLa cell would be inherited by the corresponding eukaryotic cell according to the invention. The term "polypeptide" as used herein refers to a group of molecules consisting of a plurality of amino acids connected by main chain peptide bonds. As used herein, the term also includes peptides, which consist of 30 amino acids or less. Furthermore included by the term "polypeptide" are proteins.

In a preferred embodiment, the polypeptide consists of more than 30 amino acids.

The polypeptide may be associated with further polypeptides, for example in the context of a multimeric protein. In the multimeric protein, one or more of the polypeptides may be tagged with a peptide according to the present invention.

Peptides according to the invention consist of 30 amino acids or less, preferably between 8 and 25 amino acids, more preferably between 10 and 20 amino acids. Any length in this interval is deliberately envisaged. Accordingly, peptides may consist of 11, 12, 13, 14, 15, 16, 17, 18 or 19 amino acids.

In this length range, compromising the functionality of the polypeptide to which the peptide is fused is very unlikely. The present approach is therefore distinct from the use of fusion proteins comprising fluorescent proteins which are significantly larger. The present approach is furthermore distinct from other approaches with peptide tags because it provides a cell internal read-out, thereby yielding an easily observable signal either when the tagged polypeptide is in the nucleus or when it is absent from the nucleus. This is accomplished by an oligopeptide tag, i.e., the peptide according to (c)(ii) that triggers the activity of a transcription factor which, in turn, leads to a change in the expression of a reporter gene, for example a reporter gene encoding an easily observable protein, e.g., gfp, luciferase, alkaline phosphatase, or galactosidase.

Therefore, it will deliver an easily detectable signal like fluorescence, light emission or a spectroscopically visible compound either when the tagged polypeptide is present in the nucleus or when it is absent from the nucleus. This avoids the use of microscopy to visualize nuclear localization. Furthermore, the intensity of this signal depends on the amount of tagged polypeptide in the nucleus and, hence, this method also yields quantitative information about its presence in the nucleus. Since only either the presence or the absence of the target polypeptide in the nucleus yields a signal this cell may be used in a high throughput screen for compounds interfering with nuclear transport. Therefore, it opens up a novel possibility to efficiently screen for drugs targeting diseases in which nuclear localization of a protein is altered or crucial. This pertains to drugs preventing nuclear access of polypeptide (e.g. for virus replication, or in cancer cells) or promoting nuclear access of proteins (e.g. in cancer cells), but also to drugs promoting or preventing nuclear export. Reporter gene expression correlates with the sub-cellular localization of the analyzed polypeptide (qualitative analysis) and its amount inside the nucleus can be deduced from the level of reporter gene expression (quantitative analysis). The envisaged screening method, which is further described below, makes use of the eukaryotic cell according to the invention. Being a cellular screen, it may be used in the identification of compounds affecting any cellular component involved in nuclear localization of the polypeptide of interest.

Preferred peptides being capable of binding to a transcription factor are described herein below. The means and methods described in, for example, EP1727904 can be used to generate and isolate peptides capable of binding and controlling activity of a given transcription factor.

In a preferred embodiment, said reporter gene encodes a fluorescent or chemiluminescent protein, a protein capable of generating a chemiluminescent or fluorescent compound, or a protein capable of generating a spectroscopically detectable compound. Examples of the recited fluorescent protein include green fluorescence proteins (gfp). Examples of proteins capable of generating a chemiluminescent or fluorescent compound include luciferases. Examples of proteins capable of generating a spectroscopically detectable compound include galactosidase and alkaline phosphatase. Use of such proteins, in particular for the purpose of generating a detectable readout of a biochemical or cellular essay, is established in the art.

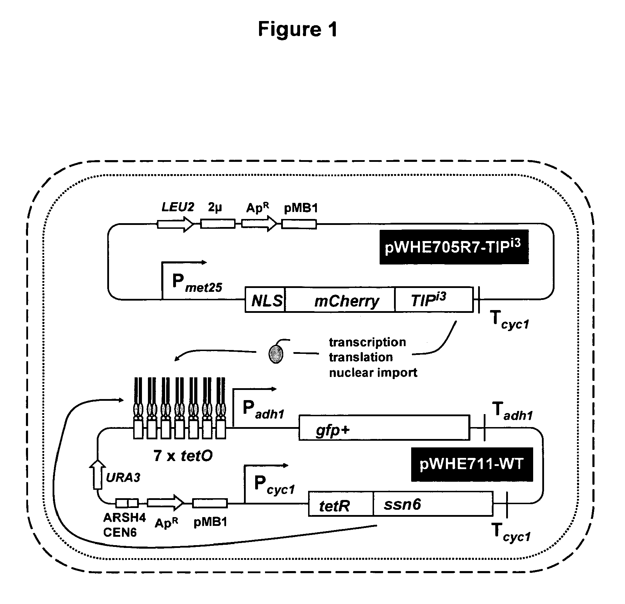

In a further preferred embodiment, said response element is a tetracycline response element. A tetracycline response element comprises one or more operator sequences which are commonly used in the art. An operator is a segment of DNA to which a repressor or activator binds. In a preferred embodiment, this operator is or comprises a sequence motif also known as *"tetO".* A tetracycline response element may comprise one or more, preferably seven *tetO* motifs; see Figure 1. Further preferred embodiments of the tetracycline response element according to the invention, i.e., specific mutants of said *tetO* motif are described further below. Preferably, the tetracycline response element is recognized and bound by a Tet repressor, a Tet repressor mutant or a reverse Tet repressor, which repressors are further detailed below. The tetracycline response element/Tet transregulator system for controlling gene expression is well-established in the art and described in, for example, Gossen and Bujard, 1992. The term "Tet transregulator" refers to a transcription factor according to the invention in which the transcription factor comprises a Tet repressor, a Tet repressor mutant or a reverse Tet repressor. The degree of gene expression of a gene controlled by an operator depends not only on the strength of the promoter, but also on the presence or absence of repressors or activators bound to the operator region.

Further constituents of the preferred tetracycline response element/Tet transregulator system according to the invention are subject of the preferred embodiments described below.

In a further preferred embodiment, said transcription factor comprises a Tet repressor or a Tet repressor mutant.

In a further preferred embodiment, said transcription factor comprises a reverse Tet repressor.

In a further preferred embodiment, said transcription factor furthermore comprises a eukaryotic transcription activation domain.

In a further preferred embodiment, said transcription factor furthermore comprises a eukaryotic transcription repression domain.

A Tet repressor is a protein which binds to a tetracycline response element and dissociates therefrom when tetracycline or a derivative thereof such as doxycycline binds to the repressor. Several Tet repressor mutants have been described in the art. Tet repressor mutants according to the invention are those mutants which deviate in their sequence from the wild-type Tet repressor sequence. A Tet repressor mutant is capable of binding to the response element according to the invention, wherein said binding is modulated by the fusion protein according to the invention. For example, preferred TetR mutants are capable of binding to their cognate DNA recognition sequence *tetO*, or to *tetO* mutants like *tetO-*4C (Helbl & Hillen, 1998), tetO-6C (Helbl et al., 1998) or *tetO*-4C5G (Krueger et al., 2007) within TRE either in the presence or absence of effector molecules like tetracycline or derivatives thereof, small molecules (Kormann et al. 2009), or TIP peptide variants. Binding of tetracycline or derivatives thereof, small molecules or TIP variants to those mutants trigger a conformational change leading either to dissociation from TRE or to binding to TRE. This applies also to fusion proteins according to the invention which comprise said TIP peptides or TIP peptide variants.

The specific Tet repressor mutant described in the enclosed examples may be used with any reporter gene and any fusion protein according to the invention. A reverse Tet repressor is a protein which binds to a tetracycline response element when doxycycline or anhydrotetracycline or other tetracyclines are bound to the reverse Tet repressor. The class of tetracyclines is abbreviated as "TET" in the following.

The Tet transregulator system has been further developed in the past by fusing transcription activation domains or transcription repression domains to a Tet repressor, a Tet repressor mutant or a reverse Tet repressor. The constructs obtained thereby are also subsumed under the term "transcription factor" as used herein. In case of a transcription activation domain being fused to a Tet repressor or a Tet repressor mutant, a tetracycline transactivator (tTA), or, when using a reverse Tet repressor as fusion partner, a reverse tetracycline transactivator (rtTA) is obtained. Binding of tetracycline or a derivative thereof (TET) to tTA renders tTA incapable of binding to the tetracycline response element (TRE), the consequence being that the gene - in the present case a reporter gene - under the control of TRE is shut off or its expression is reduced. In case of rtTA, binding of TET renders rtTA capable of binding to TRE, the consequence being that the reporter gene is turned on or its expression is increased.

If the fusion partner of a Tet repressor or a Tet repressor mutant is a transcription repression domain, a tetracycline transsilencer is obtained (tTS). If a reverse Tet repressor is used as a fusion partner, a reverse Tet transsilencer (rtTS) is obtained. Addition of TET to the former system activates expression of the reporter gene, whereas addition of TET to the latter system reduces or turns off reporter gene expression.

According to the invention, the peptide according to part (c)(ii) of the main embodiment takes the place of TET in the above-described mechanism of action of the TRE/Tet transregulator system. Accordingly, the presence in the nucleus of a fusion protein according to the invention triggers those effects in the Tet transregulator system described above as being triggered by TET.

Preference is given to those systems which, upon presence of the fusion protein according to the invention in the nucleus, increase expression of the reporter gene. Preferred are accordingly rtTA and tTS systems. However, use of tTA and rtTS systems is also deliberately envisaged. In the two latter systems, in contrast, the presence of the fusion protein according to the invention in the nucleus reduces expression of the reporter gene.

Preferred eukaryotic transcription activation domains are the VP16 domain, domains comprising one or more VP16-derived minimal activation domains "F", the activation domain of the NF-KB subunit p65, the activation domain of E2F4, the activation domain of Gal4, the p65-heat shock factor activation domain fusion, and the activation domain from MTF1. These transcription activation domains are known in the art and in part further exemplified below.

Preferred eukaryotic transcription repression domains or motifs include PLDLS, WRPW, repression domains from KRAB, CTCF, NRSF/Rest1, thyroid hormone receptor α, Ssn6 and Tup1.

In further preferred embodiments, said peptide is fused to the N-terminus or the C-terminus of said polypeptide. Preferably, said N- and C-terminal fusions are such that the sequences of the polypeptide of interest and of the polypeptide according to the invention are immediately adjacent. Alternatively, one or more intervening amino acids, such as 2 to 5, 5 to 10, 10 to 20, 20 to 30, 30 to 40, 40 to 50 or 50 to 100 amino acids are present as a linker between peptide and polypeptide.

As explained in the background section, the polypeptide of interest may be selected from a variety of vastly different proteins, wherein the selection will generally be influenced by the disease to be treated by the compounds to be identified by the screen according to the invention.

In preferred embodiments, said polypeptide of interest is selected from β-catenin, a Smad family protein, p53, MDM2, PARC, nucleophosmin, glucocorticoid receptor and viral proteins. A preferred Smad family protein is Smad 3. Envisaged viral proteins include those encoded by the genomes of double-stranded DNA viruses, single-stranded DNA viruses, double-stranded RNA viruses, positive sense single-stranded RNA viruses, negative sense single-stranded RNA viruses, positive sense single-stranded RNA viruses that replicate through a DNA intermediate and double-stranded DNA viruses that replicate through a single-stranded RNA intermediate to the extent that they localize to the nucleus or interfere with the nuclear localization of other proteins. Preferred viral proteins are those encoded by Arenaviridae, Bunyiviridae, Coronaviridae, Filoviridae, Flaviviridae, Retroviridae, Herpesviridae and Orthomyxoviridae. Further preferred viral proteins are Influenza virus proteins including RNA-dependent RNA polymerase M1, NEP, NP, NS1, Ebola virus protein VP24, and SARS virus protein ORF6. Reference is made to the discussion of viral proteins in the background section above which background section is incorporated herewith as part of the disclosure of the invention.

In preferred embodiments relating to Tet repressors, said peptide is capable of binding to and regulating the DNA-binding activity of said Tet repressor or Tet repressor mutant. Similarly, it is preferred that in relation to reverse Tet repressors said peptide is capable of binding to and regulating the DNA-binding activity of said reverse Tet repressor. It is understood that the recited Tet repressor, Tet repressor mutant or reverse Tet repressor is comprised in the transcription factor, said transcription factor optionally furthermore comprising a eukaryotic transcription activation or repression domain; see above.

In particular, the present invention in relation to Tet repressors and Tet repressor mutants uses peptides which, when bound to the transcription factor, promote dissociation thereof from the DNA. The present invention also makes use of peptides which, when bound to the transcription factor, promote binding of said transcription factor to the cognate response element, namely in conjunction with reverse Tet repressors. Both classes of peptides, those that trigger dissociation of the transcription factor from the response element and those that trigger binding of the transcription factor to the response element are further discussed below.

The following embodiments provide a structural characterization of particularly preferred peptides according to the present invention.

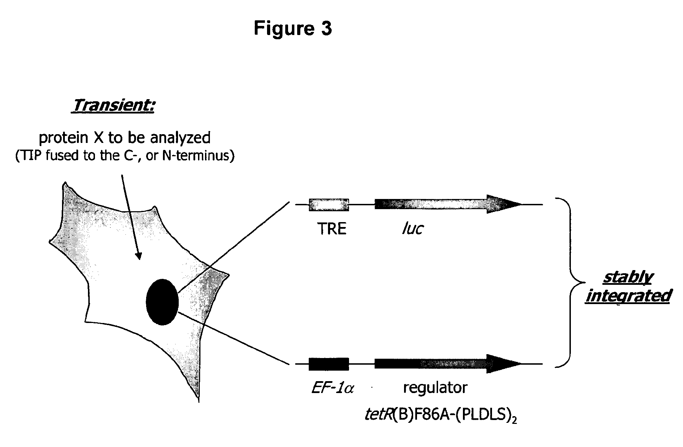

A preferred peptide which is capable of reducing the DNA-binding activity of a Tet repressor or a Tet repressor mutant is a peptide comprising or consisting of the sequence of SEQ ID NO: 1 (WTWNAYAFAAPS), herein also referred to as TlPⁱ¹. In Daam et al. 2008, the tolerance of all positions of the sequence of SEQ ID NO: 1 to substitution has been systematically explored. Reference is made to Figure 3 of Daam et al. 2008. Accordingly, further preferred peptides are those comprising or consisting of a sequence of any one of SEQ ID NOs: 2 to 12. In the sequences of SEQ ID NOs: 2 to 12, positions 1, 2 and 4 to 12 of SEQ ID NO: 1 may be varied according to the "variation" information given in the sequence listing for the respective entries.

Among peptides comprising the sequences of any one of SEQ ID NOs: 1 to 12, those peptides are preferred which consist of an aromatic residue N-terminally fused to the sequence of any one of SEQ ID NOs: 1 to 12; see SEQ ID NOs: 13 to 24. A particularly preferred aromatic residue to be fused N-terminally to the sequence of any one of SEQ ID NOs: 1 to 12 is tryptophan. A preferred embodiment of the sequence of SEQ ID NO: 13, namely the sequence WWTWNAYAFAAPS is also referred to as TIPⁱ² herein below.

A further preferred peptide, said peptide being capable of binding to and reducing the DNA-binding activity of a Tet repressor or a Tet repressor mutant is a peptide comprising or consisting of the sequence of SEQ ID NO: 25 (DDSVLAARARMWMWHW). A peptide consisting of the sequence of SEQ ID NO: 25 is also referred to as TIPⁱ³ herein below. Also, in case of the sequence of SEQ ID NO: 25, the positions which tolerate substitutions have been systematically explored. These positions are positions 1 to 5, 8 to 10 and 12. Accordingly, preferred are also peptides comprising or consisting of the sequences of SEQ ID NOs: 26 to 34. A preferred amino acid for substitution at any one of positions 1 to 5, 8 to 10 and 12 is Ala.

Among the peptides according to the invention which are capable of binding to and increasing the DNA-binding activity of a reverse Tet repressor, preference is given to peptides comprising or consisting of the sequence of SEQ ID NO: 35 (TGAIIMAVARLLPSWN). The peptide consisting of the sequence of SEQ ID NO: 35 is also referred to as TIP^{r1} herein below. Positions in the sequence of SEQ ID NO: 35 which are tolerant for substitutions are positions 1 to 3, 5, 6, 12, 14 and 16. Accordingly, further preferred peptides are those which comprise or consist of the sequence of any one of SEQ ID NO: 36 to 43. A preferred amino acid for substitution at any one of positions 1 to 3, 5, 6, 12, 14 and 16 is Ala.

Further preferred peptides according to the invention are those which are encoded by a first nucleic acid sequence which hybridizes, preferably under stringent conditions, with a second nucleic acid sequence, said second nucleic acid sequence being fully complementary to a third nucleic acid sequence encoding a peptide consisting of the sequence of any one of SEQ ID NOs: 1 to 43. It is understood that hybridization between said first and second nucleic acid sequences is brought about by at least partial complementarity in the region of said first nucleic acid which encodes the respective peptide. Such preferred peptides according to the invention are capable of binding to and regulating the DNA-binding activity of a Tet repressor, a Tet repressor mutant or a reverse Tet repressor. The terms "first nucleic acid sequence", "second nucleic acid sequence", and "third nucleic acid sequence" are used to illustrate the principle of hybridization. It is understood that the third nucleic acid according to the main embodiment, i.e., the nucleic acid encoding the fusion protein according to the invention, may comprise said first nucleic acid sequence defined above.

The term "hybridizes" as used herein refers to a pairing of one nucleic acid molecule to a second nucleic acid molecule, said two nucleic acid molecules being at least partially complementary to each other.

It is well known in the art how to perform hybridization experiments with nucleic acid molecules. Correspondingly, the person skilled in the art knows what hybridization conditions are to be used to allow for a successful hybridization in accordance with the present invention. The establishing of suitable hybridization conditions is referred to in standard text books such as Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). As stated above, in one preferred embodiment, the hybridization effected is under stringent conditions.

"Stringent hybridization conditions" refers to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization".

Also contemplated are nucleic acid molecules that hybridize to the respective target sequences according to the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, and/or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The embodiment recited herein above preferably refers to highly stringent conditions and alternatively to conditions of lower stringency.

Taken together, in conjunction with Tet repressors and Tet repressor mutants it is preferred that said peptide comprises or consists of the sequence of any one of SEQ ID NOs. 1 to 34 or that said peptide is encoded by a nucleic acid which hybridizes with a nucleic acid encoding a peptide consisting of the sequence of any one of SEQ ID NOs: 1 to 34. It is particularly preferred that said peptide comprises or consists of the sequence of SEQ ID NO: 1, 13 or 25.

Similarly, in conjunction with reverse Tet repressors it is preferred that said peptide comprises or consists of the sequence of SEQ ID NOs. 35 to 43 or that said peptide is encoded by a nucleic acid which hybridizes with a nucleic acid encoding a peptide consisting of the sequence of any one of SEQ ID NOs: 35 to 43. It is particularly preferred that said peptide comprises or consists of the sequence of SEQ ID NO: 35.

Further preferred peptides according to the invention are those which comprise or consist of an amino acid sequence, said sequence exhibiting at least 75% sequence identity to the sequence of any one of SEQ ID NOs: 1 to 43. It is understood that said sequence identity occurs over the entire length of said sequence of any one of SEQ ID NOs: 1 to 43. In preferred embodiments sequence identity is at least 80%, at least 85%, or at least 90%. Further preferred are sequence identities above 90% such as 91%, 92%, 93%, 94%, 95%, 96%, 97%, 98%, 99% as well as complete identity (100% sequence identity). Peptides according to the invention which exhibit the above-described levels of sequence identity are capable of binding to and regulating the DNA-binding activity of a Tet repressor, a Tet repressor mutant or a reverse Tet repressor.

It is understood that the above-described peptides according to the invention, by binding to said Tet repressor, said Tet repressor mutant or said reverse Tet repressor, bind to a transcription factor according to the invention, said transcription factor comprising a Tet repressor, a Tet repressor mutant or a reverse Tet repressor.

Table 4 shows several embodiments of the invention indicating how it can be used with TetR based transcription factors to exemplify its use with any transcription factor active in a eukaryotic cell.

**Table 4: Collection of transcription inducing peptides (TIP) used in the present application**

| ***Peptide tag*** | ***Sequence*** | ***Transregulator specificity and type*** | ***Applied in (organism)*** |
|---|---|---|---|
| TIPⁱ¹ | WTWNAYAFAAPS (SEQ ID NO: 1) | TetR-(PLDLS)_{2;} tTS | human cell line |
| TIPⁱ² | WWTWNAYAFAAPS (SEQ ID NO: 13) | TetR(F86A)-(PLDLS)₂; tTS | human cell line |
| TIPⁱ³ | DDSVLAARARMWMWHW (SEQ ID NO: 25) | TetR-Ssn6; tTS | *S. cerevisiae* |
| | | TetR-(PLDLS)₂₁ tTS | human cell line |
| TIP^{r1} | TGAIIMAVARLLPSWN (SEQ ID NO: 35) | revTetR-(F)₃; rtTA | human cell line |

| | | | |
|---|---|---|---|
| TIPⁱ : inducer, induction of wt TetR TIP^{r} : corepressor, corepression of rev TetR | | | |

In a further preferred embodiment of the invention said cell is a S. *cerevisiae* cell, an invertebrate cell or a vertebrate cell. Preferred invertebrate cells include Schneider cells. Preferred vertebrate cells include embryonic stem (ES) cells and induced pluripotent stem (iPS) cells and ES- or iPS cell-derived cell lines, as well as A549 Alveolar epithelial cells, 293 cells, and human cancer derived cells lines such as HeLa, Jurkat and PC3 cells. In a preferred embodiment, said PC3 cell overexpresses osteopontin.

The present invention furthermore relates to a method of determining the presence of a polypeptide of interest in the nucleus of a eukaryotic cell, said method comprising: (a) culturing the cell according to the invention; and (b) determining whether the reporter gene comprised in said cell is expressed.

In principle, said determination may be effected either at the mRNA or at the protein level by using means and methods well-known in the art. Methods for the determination of mRNA expression levels include real-time PCR, Northern blotting and hybridization on microarrays or DNA chips equipped with one or more probes or probe sets specific for transcripts of the reporter gene.

Alternatively or in addition, the expression level to be determined is the polypeptide expression level. Methods of quantifying a particular protein (in this case a reporter protein) may rely on specific binding, e.g. of antibodies. Western blotting combines separation of a mixture of proteins by electrophoresis and specific detection with antibodies. Electrophoresis may be multidimensional such as two-dimensional. Usually, polypeptides are separated in 2D-electrophoresis by their apparent molecular weight along one dimension and by their isoelectric point along the other direction.

Preferred means and methods for determining expression of the reporter gene are described further below.

When using a tTS or an rtTA system, expression of the reporter gene is indicative of the presence of said polypeptide of interest in the nucleus of said eukaryotic cell. When using an rtTS or a tTA system, absence of expression of the reporter gene is indicative of the presence of said polypeptide of interest in the nucleus of said eukaryotic cell. More generally speaking, any response element/transcription factor combination which activates expression of the reporter gene upon binding of the peptide according to the invention may be used instead of a tTS or rtTA system. Similarly, any response element/transcription factor combination which turns off expression of the reporter gene upon binding of the peptide according to the invention may be used instead of rtTS or tTA systems.

Also provided by the present invention is a method of identifying a compound capable of altering the nuclear localization of a polypeptide of interest, said method comprising the method as defined above, wherein said culturing is effected in the presence and, independently, in the absence of a test compound, respectively, and wherein a difference in expression of said reporter gene in presence of the test compound as compared to the absence of said test compound is indicative of the test compound being a compound capable of altering the nuclear localization of said polypeptide of interest.

The term "altering" refers to interfering with nuclear localization of a polypeptide of interest and is understood to comprise both "increasing" and "decreasing". Determining whether the reporter gene is expressed is preferably performed quantitatively.

Test compounds may be any compounds, preferably low molecular weight organic compounds. The term "low molecular weight" as used in this context refers to compounds having a molecular weight below 2,000, preferably below 1,000, and more preferred below 800, 700, 600 or 500 atomic mass units.

Preferably, said method is performed in high-throughput format. High-throughput assays, independently of being biochemical, cellular or other assays, generally may be performed in wells of microtiter plates, wherein each plate may contain 96, 384 or 1536 wells. Handling of the plates, including incubation at temperatures other than ambient temperature, and bringing into contact of test compounds with the assay mixture is preferably effected by one or more computer-controlled robotic systems including pipetting devices. In case large libraries of test compounds are to be screened and/or screening is to be effected within short time, mixtures of, for example 10, 20, 30, 40, 50 or 100 test compounds may be added to each well. In case a well exhibits biological activity, said mixture of test compounds may be de-convoluted to identify the one or more test compounds in said mixture giving rise to said activity.

In preferred embodiments of the methods according to the invention, said reporter gene encodes a fluorescent protein, a protein capable of generating a chemiluminescent or fluorescent compound, or a protein capable of generating a spectroscopically detectable compound and said determining is effected by determining the amount of fluorescence generated by said fluorescent protein or the amount of said chemiluminescent, fluorescent, or spectroscopically detectable compound. In other words, said determining is preferably effected quantitatively.

The present invention furthermore relates to a use of the cell according to the invention or of the third nucleic acid as defined in conjunction with the cell according to the invention for determining the presence of said polypeptide of interest in the nucleus.

The present invention furthermore relates to a use of the cell according to the invention or of the third nucleic acid as defined in conjunction with the cell according to the invention for identifying a compound capable of interfering with the nuclear localization of said polypeptide of interest.

The present invention furthermore relates to a use of the third nucleic acid as defined in the invention for transfecting, nucleofecting or transducing a eukaryotic cell.

Furthermore provided is a kit for transfecting, nucleofecting or transducing a eukaryotic cell and/or for performing the method as defined herein above, said kit comprising first, second and third nucleic acids as defined in conjunction with the cell according to the invention.

As detailed herein above, one or more nucleic acids selected from said first, second and third nucleic acids may be realized by a single nucleic acid. For example, a single nucleic acid species may (i) comprise a reporter gene under the control of a response element and (ii) a gene encoding a transcription factor, said transcription factor being capable of binding to said response element, thereby controlling expression of said reporter gene. In any case, the one or more constituents of the kit according to the invention may be provided in one or more separate vials. In a preferred embodiment, each nucleic acid is provided in a separate vial. Said one or more vials may be contained in a container, the container optionally furthermore comprising a manual with instructions for performing any of the methods or uses according to the present invention.

The figures show:
**Figure 1****:** Plasmid encoded reporter system to analyze TIP-mediated TetR induction in *Saccharomyces cerevisiae.*
   A schematic overview of the plasmid encoded reporter system is shown. The plasmid pWHE705R7 encodes for the Pₘₑₜ₂₅ controlled fluorescent protein mCherry fused to the TIP variant TIPⁱ³. In the absence of methionine, the mCherry-TIP fusion is expressed and can interact with the constitutively expressed TetR-Ssn6 encoded by pWHE711. TetR-Ssn6 regulates the expression of a *gfp+* reporter gene via a Tet-responsive promoter located on the same plasmid.

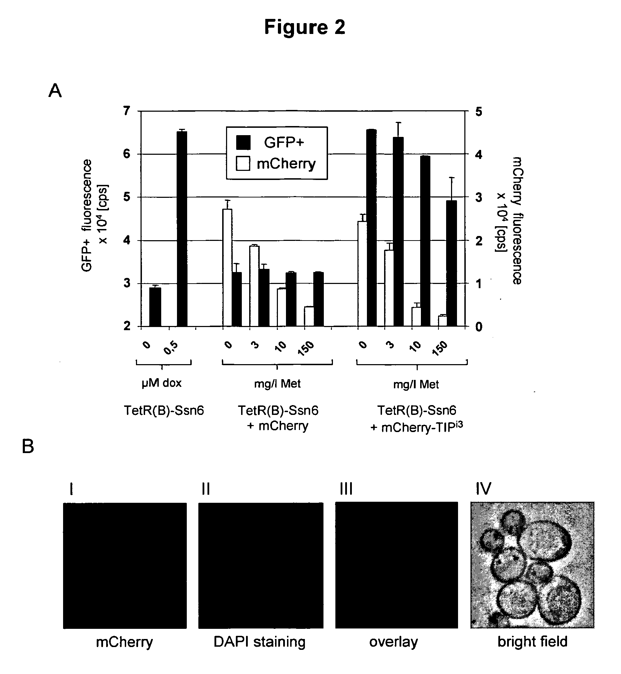

**Figure 2****:** TIP-mediated induction of TetR in *Saccharomyces cerevisiae* and nuclear localization of the TIP-tagged protein.
   A. Induction of TetR(B)-Ssn6 by doxycycline and the TIP variant TIPⁱ³ in S. *cerevisiae.* Fluorescence of TetR-controlled GFP+ is shown on the left-hand axis and bars are depicted in black. TetR(B) is fused to the yeast repression domain Ssn6. Fluorescence correlating with expression of mCherry and mCherry-TIPⁱ³, respectively, is shown on the right-hand axis and bars are depicted in grey. Expression of the mCherry proteins is under control of a methionine-responsible promoter, and is varied by alterering the methionine concentration.
   B. Localization of Tip fusion proteins using fluorescence microscopy. I: Fluorescence of the mCherry-TIPⁱ³ fusion protein. II: Visualization of the nuclei by DAPI staining. III: Overlay of I and II. IV: Microscopic bright field image of analyzed cells.
**Figure 3****:** Double-stable HeLa cell line to analyze nucleo-cytoplasmic transport.
   A schematic of the cell line HeLa HF1-3-*luc*/*tTS* is shown. The luciferase *(luc)* gene downstream of a Tet-responsive element (TRE) is stably integrated, as well as the trans-silencer *tetR*(B)F86A-(PLDLS)₂ which is constitutively expressed via the EF-1α promoter. Binding of this tTS variant leads to repression of *luc* expression. Proteins to be analyzed are cloned into eukaryotic expression plasmids as either C-, or N-terminal TIP fusions and are transiently transfected.

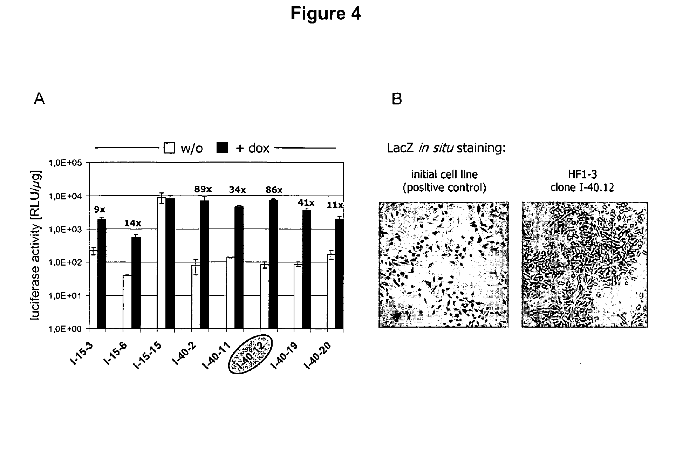

**Figure 4****:** Properties of HeLa HF1-3-luc/tTS cell clones.
   **A.** Regulatory window permitted by various HF1*-*3*-luc*/*tTS* cell clones. *luc* activity was measured in the absence (grey bars) and presence of 2 µM doxycycline (black bars). Factors of regulation are indicated above the respective bars.
   **B.** LacZ *in situ* staining of the cell line 1-40.12. The integration locus of the initial cell line is characterized by a single FRT site, a hygromycin resistance cassette and a *lacZ* gene encoding for β-galactosidase. Addition of an Xgal staining solution causes blue colored cells. Flp recombinase mediated integration of the tTS variant leads to a disruption of *lacZ* and to the disappearance of the blue staining proofing homogeneity of the cell line.

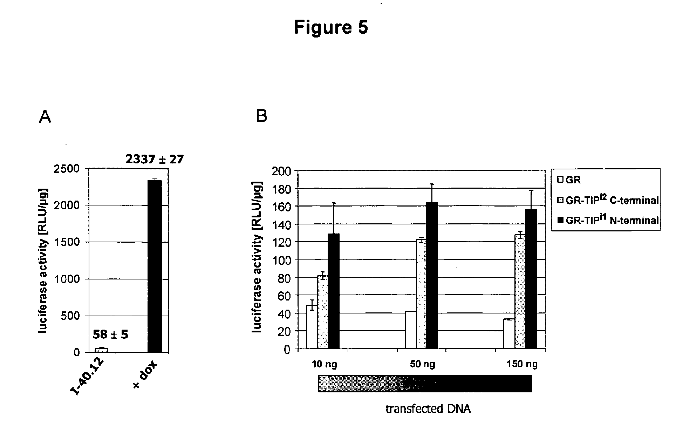

**Figure 5****:** Induction of *luc* expression mediated by TIP-tagged glucocorticoid receptor variants in HeLa cells.
   **A.** *luc* expression of the cell line HeLa HF1-3-*lucltTS* l-40.12 in the absence (grey bar) and presence of 2 µM doxycycline (black bar). Luciferase activity in RLU/µg is indicated above the respective bars.
   **B.** *luc* expression after transient transfection of expression plasmids encoding for various glucocorticoid receptor variants (GR). GR wt is indicated with white bars, C-terminal TIPⁱ² fusions with grey bars and N-terminal TIPⁱ¹ fusions with black bars. DNA amounts ranging from 10 to 150 ng were used for transient transfection experiments.

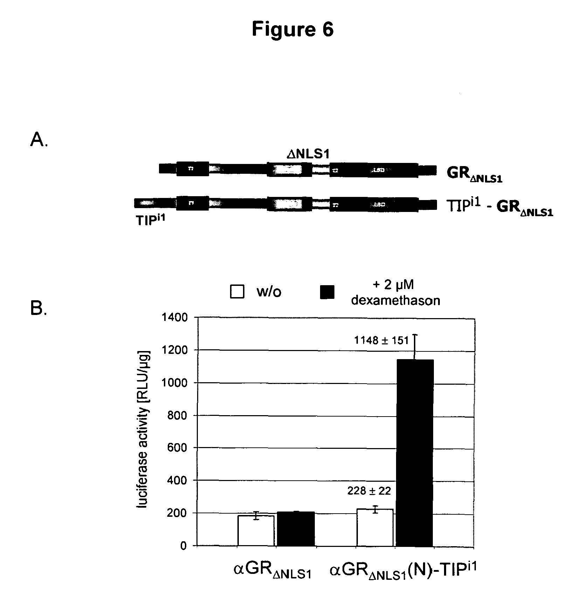

**Figure 6****:** Detection of dexamethasone-dependent nuclear transport of glucocorticoid receptor variants with mutated NLS1.
   **A.** Molecular architecture of glucocorticoid receptor variants with mutated NLS1. The receptor variant GR_{ΔNLS1} is characterized by the mutations K513N, K514N and K515N within nuclear localization sequence 1, designated "ΔNLS1", overlapping the DNA-binding domain (designated "DBD"). The ligand binding domain is designated "LBD", the activation domains "τ1" and "τ2". TIPⁱ¹-GR_{ΔNLS1}, in addition to the aforementioned variant, is characterized by an N-terminal TIPⁱ¹ fusion.
   **B.** *luc* expression after transient transfection of expression plasmids encoding for glucocorticoid receptor variants αGR_{ΔNLS1} and αGR_{ΔNLS1}(N)-TIPⁱ¹ in HeLa HF1.3-luc/tTS. Luciferase activity in the absence of dexamethasone is indicated with grey bars, in the presence of 2 µM dexamethasone with black bars. Luciferase activity obtained for αGR_{ΔNLS1}(N)-TIPⁱ¹ in the presence and absence of the artificial hormone is indicated next to the bars. Transient transfection experiments were performed using special medium treated to reduce serum-borne hormones.

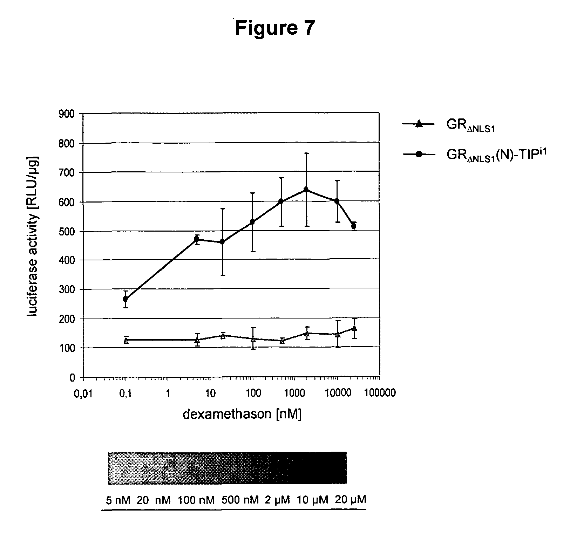

**Figure 7****:** Analysis of nuclear glucocorticoid receptor variants at various dexamethasone concentrations.
   *luc* expression after transient transfection of expression plasmids encoding for glucocorticoid receptor variants αGR_{ΔNLS1} (circles) and αGR_{ΔNLS1}(N)-TIPⁱ¹ (triangles) in HeLa HF1.3-luc/tTS. Dexamethasone concentrations were varied from 5 nM to 20 µM as indicated underneath the graph. Transient transfection experiments were performed using special medium treated to reduce serum-borne hormones.

The following example illustrates the invention but should not be construed as being limiting.

### Example 1

### Scoring nuclear localization of proteins in Yeast

*Saccharomyces cerevisiae* was selected as a model eukaryote to exemplify the screening system for nuclear transport. To be able to demonstrate nuclear localization, we fused the variant TIPⁱ³ (see Table 4 above) to the C-terminus and an SV40 nuclear localization sequence (NLS) to the N-terminus of the red fluorescent protein mCherry (see Figure 1). This fusion protein was expressed under transcriptional control of the S. *cerevisiae* met25 promoter. This allows downregulation of its expression by increasing the methionine concentration in the growth medium. The reporter system consists of TetR(B) to which the yeast transcription repression domain Ssn6 (Schultz et al., 1990) was C-terminally fused and placed under transcriptional control of the *S. cerevisiae cyc1* promoter, leading to constitutive expression of the transregulator TetR-Ssn6 at a low level. We then inserted seven consecutive *teto* boxes upstream of the *adh1* promoter originating from *Schizosaccharomyces pombe* and used this for Tet-controlled *gfp+* expression. This construct is repressed in the presence of TetR-Ssn6. In the absence of methionine, the NLS-mCherry-TIPⁱ³ fusion protein is expressed and results in significant induction of *gfp+* expression (see Figure 2A). Expression of the NLS-mCherry without the TIP tag results only in basal expression of *gfp*+*.* The nuclear localization of the NLS-mCherry-TIPⁱ³ protein was determined by fluorescence microscopy and is shown in Figure 2B. These data demonstrate unambiguously that reporter gene expression is induced with increasing mCherry-TIPⁱ³ fusion protein amounts, and hence, that the presence of a TIP fusion protein in the nucleus is detected by *gfp*+ expression. This yeast strain can be used for HTS to detect lead structures of compounds which prevent the accumulation of any protein, here represented by mCherry, in the nucleus.

### Example 2

### Scoring nuclear localization of proteins in human cell lines

For proof of principle of the nuclear localisation screen in mammalian cells, we used the well characterized nucleo-cytoplasmic shuttling of the glucocorticoid receptor (GR) in the human HeLa cell line. We started to construct the screening cell line from HeLa HF1-3 containing a single Flp recognition target site (FRT) for specific integration via Flp recombinase (Sauer, 1994, Drüppel 2004). First, we integrated a Tet-responsive *luc* gene randomly into the genome and screened for clones showing high and Tet-regulatable *luc* expression by transiently transfecting a construct encoding for rtTA, consisting of a TetR(B) variant fused to the minimal activation domain FFF (Urlinger et al., 2000) from the VP16 domain (Gossen et al., 1992). A clone from those displaying the highest factor of regulation was chosen for stable integration of a constitutively expressed tTS construct consisting of TetR(B)F86A fused to two repeats of the eukaryotic repression domain PLDLS (Gray et al., 1994), (Schaeper et al., 1995). The resulting candidates were analyzed for their regulatory properties and cellular homogeneity. The cell line selected for further use is depicted in Figure 3. Its regulation of *luc* expression is demonstrated in Figure 4. The TetR moiety of the integrated tTS is the variant TetR(B)F86A, which is more sensitive towards TIP^{i1/i2}-mediated induction than wt TetR (Klotzsche et al., 2007). To demonstrate that luciferase expression is indeed induced by a TIP fusion protein we transiently transfected an expression plasmid encoding for the glucocorticoid receptor (GR), either with an N- or a C-terminal TIP tag, or without any tag. The results shown in Figure 5B clearly demonstrate for the first time induction of a reporter gene mediated by a protein fused to TIP in a human cell line.

It has been shown that high expression of GR, which contains two NLS elements (shown in Figure 6A), NLS1 and a less well defined NLS2 located within the ligand binding domain, can lead to constitutive nuclear localization. Therefore, we used the mutant GR-K513N-K514N-K515N (GR_{ΔNLS1}) (Savory et al., 1999), in which three lysine residues within NLS1 are replaced by asparagine. As a consequence, nuclear transport of GR_{ΔNLS1} completely relies on NLS2 which is masked by complex formation with chaperones in the absence of hormone, and hence, stays in the cytoplasm. Binding of glucocorticoid hormones or analogs like dexamethasone to GR dissociates this complex (Madan and DeFranco, 1993), (Sackey et al., 1996). As a result, GR can enter the nucleus only after addition of such compounds. The GR_{ΔNLS1} mutant was cloned into eukaryotic expression plasmids with and without N-terminal TIPⁱ¹ fusion. Transient transfection experiments were performed using medium treated to reduce serum-borne hormones. Figure 6 illustrates that transfection with the GR_{ΔNLS1}(N)-TIPⁱ¹ encoding plasmid leads only in the presence of dexamethasone to a 5-fold increase of luciferase expression, while it remains unchanged in the absence of the hormone. The maximal luciferase expression is observed at a concentration of 2 µM dexamethasone. Expression of GR_{ΔNLS1} without TIP does not lead to an increase in luciferase expression in the presence or absence of dexamethasone. This result shows unambiguously that the presence of the fusion protein TIPⁱ¹-GR_{ΔNLS1} in the nucleus of the indicator cell line leads to luciferase expression. The well-known effect of dexamethasone to enable GR to enter the nucleus of the cell therefore validates the constructed screening cell line and teaches clearly how novel compounds with the same effect on either the TIP-GR_{ΔNLS1} or on any other target protein-TIP fusion can be identified by this screen. Luciferase as a reporter may be replaced by other reporters like gfp or other enzymes.

### Further references

Agha-Mohammadi, S., O'Malley, M., Etemad, A., Wang, Z., Xiao, X. and Lotze, M. T. (2004). Second-generation tetracicline-regulatable promoter: repositioned tet operators optimize transactivator synergy while shorter minimal promoter offers tight basal leakiness. J Gene Med, 6, 817-28.
Baltimore, D. (1971) Expression of animal virus genomes. Bacteriological Reviews, 35, 235-41
Baiter, M. (2000). Emerging diseases. On the trail of Ebola and Marburg viruses. Science 290, 923-5.
Boulo, S., Akarsu, H., Ruigrok, R. W. and Baudin, F. (2007). Nuclear traffic of influenza virus proteins and ribonucleoprotein complexes. Virus Res 124, 12-21.
Chang, Y., Cesarman, E., Pessin, M. S., Lee, F., Culpepper, J., Knowles, D. M. and Moore, P. S. (1994). Identification of herpesvirus-like DNA sequences in AIDS-associated Kaposi's sarcoma. Science. 266, 1865-9.
Cheng, V. C., Lau, S. K., Woo, P. C. and Yuen, K. Y. (2007). Severe acute respiratory syndrome coronavirus as an agent of emerging and reemerging infection. Clin Microbiol Rev 20, 660-94.
Crawford, D. H. (2001). Biology and disease associations of Epstein-Barr virus. Philos Trans R Soc Lond B Biol Sci. 29, 356, 461-73.
Daam, J., Mehdaoui, K., Klotzsche, M., Pfleiderer, K., Berens, C. and Hillen, W. (2008). Functionally important residues of the Tet repressor inducing peptide TIP determined by a complete mutational analysis. Gene 423, 201-06.
Dahl, E., Kristiansen, G., Gottlob, K., Klaman, I., Ebner, E., Hinzmann, B., Hermann, K., Pilarsky, C., Durst, M., Klinkhammer-Schalke, M. et al. (2006). Molecular profiling of laser-microdissected matched tumor and normal breast tissue identifies karyopherin alpha2 as a potential novel prognostic marker in breast cancer. Clin Cancer Res 12, 3950-60.
de Jong, M. D., Tran, T. T., Truong, H. K., Vo, M. H., Smith, G. J., Nguyen, V. C., Bach, V. C., Phan, T. Q., Do, Q. H., Guan, Y. et al. (2005). Oseltamivir resistance during treatment of influenza A (H5N1) infection. N Engl J Med 353, 2667-72.
Drüppel, L. (2004). Characterisation of the multivalent transcription factor CTCF. PhD-thesis, Friedrich-Alexander Universität Erlangen-Nürnberg.
Dyer, R. B. and Herzog, N. K. (1995). Isolation of intact nuclei for nuclear extract preparation from a fragile B-lymphocyte cell line. Biotechniques 19, 192-5.
Ellis, B. R. and Barrett, A. D. (2008). The enigma of yellow fever in East Africa. Rev Med Virol 18, 331-46.
Falini, B., Bolli, N., Liso, A., Martelli, M. P., Mannucci, R., Pileri, S. and Nicoletti, I. (2009). Altered nucleophosmin transport in acute myeloid leukaemia with mutated NPM1: molecular basis and clinical implications. Leukemia 23, 1731-43.
Feldherr, C. M. (1962). The nuclear annuli as pathways for nucleocytoplasmic exchanges. J Cell Biol 14, 65-72.
Feldherr, C. M., Kallenbach, E. and Schultz, N. (1984). Movement of a karyophilic protein through the nuclear pores of oocytes. J Cell Biol 99, 2216-22.
Feldherr, C. M. and Marshall, J. M., Jr. (1962). The use of colloidal gold for studies of intracellular exchanges in the ameba Chaos chaos. J Cell Biol 12, 640-5.
Frieman, M., Yount, B., Heise, M., Kopecky-Bromberg, S. A., Palese, P. and Baric, R. S. (2007). Severe acute respiratory syndrome coronavirus ORF6 antagonizes STAT1 function by sequestering nuclear import factors on the rough endoplasmic reticulum/golgi membrane. J Virol 81, 18, 9812-9824.
Geisbert, T. W. and Jahrling, P. B. (2004). Exotic emerging viral diseases: progress and challenges. Nat Med 10, S110-21.
Go W. Y. and Ho S. N.(2002). Optimization and direct comparison of the dimerizer and reverse tet transcriptional control systems. J Gene Med, 4(3):258-70
Gopalkrishnan, R. V. Christiansen, K. A., Goldstein, N. I., DePinho, R. A. and Fisher, P. B. (1999). Use of the human EF-1α promoter for expression can significantly increase success in establishing stable cell lines with consistent expression: a study using tetracycline-inducible system in human cancer cells. Nucleic Acids Res 27, 4775-82.
Gossen, M. and Bujard, H. (1992). Tight control of gene expression in mammalian cells by tetracycline-responsive promotors. Proc Natl Acad Sci USA 89, 5547-51
Gray, S., Szymanski, P. and Levine, M. (1994). Short-range repression permits multiple enhancers to function autonomously within a complex promoter. Genes Dev 8, 1829-38.
Grisendi, S., Mecucci, C., Falini, B. and Pandolfi, P. P. (2006). Nucleophosmin and cancer. Nat Rev Cancer 6, 493-505.
Halder, S. K., Rachakonda, G., Deane, N. G. and Datta, P. K. (2008). Smad7 induces hepatic metastasis in colorectal cancer. Br J Cancer 99, 957-65.
Helbl, V., and Hillen, W. (1998). Stepwise selection of TetR variants recognizing tet operator 4C with high affinity and specificity. J Mol Biol. 276, 313-8.
Helbl, V., Tiebel, B., and Hillen, W. Stepwise selection of TetR variants recognizing tet operator 6C with high affinity and specificity. J Mol Biol. 276, 319-24.
**Hillen, W., Klotzsche, M., Berens C.** (2005). "Peptide-based method for monitoring gene expression in a host cell" - Publication Number WO/2005/093075, International Application Number PCT/EP2005/003126
Hoffmann, A. Villalba, M., Journot, L. and Spengler, D. (1997) A novel tetracycline-dependent expression vector with low basal expression and potent regulatory properties in various mammalian cell lines. Nucleic Acids Res 25, 1078-9.
Kau, T. R., Way, J. C. and Silver, P. A. (2004). Nuclear transport and cancer: from mechanism to intervention. Nat Rev Cancer 4, 106-17.
Kemler, I., Whittaker, G. and Helenius, A. (1994). Nuclear import of microinjected influenza virus ribonucleoproteins. Virology 202, 1028-33.
Khanna, M., Kumar, P., Choudhary, K., Kumar, B. and Vijayan, V. K. (2008). Emerging influenza virus: a global threat. J Biosci 33, 475-82*.*
Kloth, J. N., Kenter, G. G., Spijker, H. S., Uljee, S., Corver, W. E., Jordanova, E. S., Fleuren, G. J. and Gorter, A. (2008). Expression of Smad2 and Smad4 in cervical cancer: absent nuclear Smad4 expression correlates with poor survival. Mod Pathol 21, 866-75.
Klotzsche, M., Berens, C. and Hillen, W. (2005). A peptide triggers allostery in tet repressor by binding to a unique site. J Biol Chem 280, 24591-9.
Klotzsche, M., Goeke, D., Berens, C. and Hillen, W. (2007). Efficient and exclusive induction of Tet repressor by the oligopeptide Tip results from co-variation of their interaction site. Nucleic Acids Res 35, 3945-52.
Kormann, C., Pimenta, I., Löber, S., Wimmer, C., Lanig, H., Clark, T., Hillen, W., and Gmeiner, P. (2009). Diarylpropane-1,3-dione derivatives as TetR-inducing tetracycline mimetics: Synthesis and biological investigations. Chembiochem. 10, 2924-33.
Krueger, C., Danke, C., Pfleiderer, K., Schuh, W., Jäck, H.M., Lochner, S., Gmeiner, P., Hillen, W. and Berens, C. (2006). A gene regulation system with four distinct expression levels. J Gene Med, 8, 1037-47.
Krueger, M., Scholz, O., Wisshak, S. and Hillen, W. (2007) Engineered Tet repressors with recognition specificity for the tetO-4C5G operator variant. Gene 404, 93-100.
Lane, D. P. (1992). Cancer. p53, guardian of the genome. Nature 358, 15-6.
Le, Q. M., Kiso, M., Someya, K., Sakai, Y. T., Nguyen, T. H., Nguyen, K. H., Pham, N. D., Ngyen, H. H., Yamada, S., Muramoto, Y. et al. (2005). Avian flu: isolation of drug-resistant H5N1 virus. Nature 437, 1108.
Leyssen, P., De Clercq, E. and Neyts, J. (2008). Molecular strategies to inhibit the replication of RNA viruses. Antiviral Res 78, 9-25.
Lisenbee, C. S., Karnik, S. K. and Trelease, R. N. (2003). Overexpression and mislocalization of a tail-anchored GFP redefines the identity of peroxisomal ER. Traffic 4, 491-501.
Lu, S., Lee, J., Revelo, M., Wang, X. and Dong, Z. (2007). Smad3 is overexpressed in advanced human prostate cancer and necessary for progressive growth of prostate cancer cells in nude mice. Clin Cancer Res 13, 5692-702.
Luu, H.H. et al. (2004). Wnt/β-catenin signaling pathway as a novel cancer drug target. Curr Cancer Drug Targets 4, 653-671.
Luckner, S. R., Klotzsche, M., Berens, C., Hillen, W. and Muller, Y. A. (2007). How an agonist peptide mimics the antibiotic tetracycline to induce Tet-repressor. J Mol Biol 368, 780-90.
Madan, A. P. and DeFranco, D. B. (1993). Bidirectional transport of glucocorticoid receptors across the nuclear envelope. Proc Natl Acad Sci U S A 90, 3588-92.
Min, J. Y. and Krug, R. M. (2006). The primary function of RNA binding by the influenza A virus NS1 protein in infected cells: Inhibiting the 2'-5' oligo (A) synthetase/RNase L pathway. Proc Natl Acad Sci USA 103, 7100-5.
Momand, J., Wu, H. H. and Dasgupta, G. (2000). MDM2--master regulator of the p53 tumor suppressor protein. Gene 242, 15-29.
Morin, P.J. (1999). β-catenin signaling and cancer. Bioessays 21, 1021-30.
Neumann, G., Hughes, M. T. and Kawaoka, Y. (2000). Influenza A virus NS2 protein mediates vRNP nuclear export through NES-independent interaction with hCRM1. EMBO J 19, 6751-8.
Newlands, E. S., Rustin, G. J. and Brampton, M. H. (1996). Phase I trial of elactocin. Br J Cancer 74, 648-9**.**
Nikolaev, A. Y. and Gu, W. (2003). PARC: a potential target for cancer therapy. Cell Cycle 2, 169-71.
Radu, A., Moore, M. S. and Blobel, G. (1995). The peptide repeat domain of nucleoporin Nup98 functions as a docking site in transport across the nuclear pore complex. Cell 81, 215-22.
Reid, S. P. Valmas, C., Martinez, O., Sanchez, F. M. and Basler, C. F. (2007). Ebola virus VP24 proteins inhibit the interaction of NPI-1 subfamily karyopherin α proteins with activated STAT1. J Virol 81, 13469-77.
Robertson, B. W. and Chellaiah, M. A. (2009). Osteopontin induces β-catenin signaling through activation of Akt in prostate cancer cells. Exp Cell Res 316, 1-11.
Rout, M. P., Aitchison, J. D., Suprapto, A., Hjertaas, K., Zhao, Y. and Chait, B. T. (2000). The yeast nuclear pore complex: composition, architecture, and transport mechanism. J Cell Biol 148, 635-51.
Sackey, F. N., Hache, R. J., Reich, T., Kwast-Welfeld, J. and Lefebvre, Y. A. (1996). Determinants of subcellular distribution of the glucocorticoid receptor. Mol Endocrinol 10, 1191-205.
Sauer, B. (1994). Site-specific recombination: developments and applications. Curr Opin Biotechnol 5, 521-7.
Savory, J. G., Hsu, B., Laquian, 1. R., Giffin, W., Reich, T., Hache, R. J. and Lefebvre, Y. A. (1999). Discrimination between NL1- and NL2-mediated nuclear localization of the glucocorticoid receptor. Mol Cell Biol 19, 1025-37.
Schaeper, U., Boyd, J. M., Verma, S., Uhlmann, E., Subramanian, T. and Chinnadurai, G. (1995). Molecular cloning and characterization of a cellular phosphoprotein that interacts with a conserved C-terminal domain of adenovirus E1A involved in negative modulation of oncogenic transformation. Proc Natl Acad Sci U S A 92, 10467-71.
Schultz, J., Marshall-Carlson, L. and Carlson, M. (1990). The N-terminal TPR region is the functional domain of SSN6, a nuclear phosphoprotein of Saccharomyces cerevisiae. Mol Cell Biol 10, 4744-56.
Szulc, J., Wiznerowicz, M., Sauvain, M. O., Trono, D. and Aebischer, P. A (2006) A versatile tool for conditional gene expression and knockdown. Nat Methods. 3, 109-16.
Suzuki, Y. and Craigie, R. (2007). The road to chromatin - nuclear entry of retroviruses. Nat Rev Microbiol 5, 187-96.
Terry, L. J., Shows, E. B. and Wente, S. R. (2007). Crossing the nuclear envelope: hierarchical regulation of nucleocytoplasmic transport. Science 318, 1412-6.
Thomas, C. L. and Maule, A. J. (2000). Limitations on the use of fused green fluorescent protein to investigate structure-function relationships for the cauliflower mosaic virus movement protein. J Gen Virol 81, 1851-5.
Urlinger, S., Baron, U., Thellmann, M., Hasan, M. T., Bujard, H. and Hillen, W. (2000). Exploring the sequence space for tetracycline-dependent transcriptional activators: novel mutations yield expanded range and sensitivity. Proc Natl Acad Sci USA 97, 7963-8.
van der Watt, P. J., Maske, C. P., Hendricks, D. T., Parker, M. I., Denny, L., Govender, D., Birrer, M. J. and Leaner, V. D. (2009). The Karyopherin proteins, Crm1 and Karyopherin beta1, are overexpressed in cervical cancer and are critical for cancer cell survival and proliferation. Int J Cancer 124, 1829-40.
Wang, P., Palese, P. and O'Neill, R. E. (1997). The NPI-1/NPI-3 (karyopherin alpha) binding site on the influenza a virus nucleoprotein NP is a nonconventional nuclear localization signal. J Virol 71, 1850-6.
Whitehouse, C. A. (2004). Crimean-Congo hemorrhagic fever. Antiviral Res 64, 145-60.
Winnepenninckx, V., Lazar, V., Michiels, S., Dessen, P., Stas, M., Alonso, S. R., Avril, M. F., Ortiz Romero, P. L., Robert, T., Balacescu, O. et al. (2006). Gene expression profiling of primary cutaneous melanoma and clinical outcome. J Natl Cancer Inst 98, 472-82.
Yao, F., Svenjö, T., Winkler, T., Lu, M., Eriksson, C., Eriksson, E. (1998). Tetracycline Repressor, tetR, rather than the tetR-mammalian cell transcription factor fusion derivatives, regulates inducible gene expression in mammalian cells. Hum. Gene Ther. 9, 1939-50.
Zhirnov, O. P. (1992). Isolation of matrix protein M1 from influenza viruses by acid-dependent extraction with nonionic detergent. Virology 186, 324-30.
Zhong, H., Shio, H. and Yaseen, N. R. (2006). Ultrastructural nuclear import assay. Methods 39, 309-15.

## Claims

1. A eukaryotic cell comprising:
(a) a first nucleic acid comprising a reporter gene under the control of a response element;
(b) a second nucleic acid comprising a gene encoding a transcription factor, said transcription factor being capable of binding to said response element, thereby controlling expression of said reporter gene; and
(c) a third nucleic acid encoding a fusion protein, said fusion protein comprising:
(i) a polypeptide of interest; and
(ii) a peptide being capable of binding to said transcription factor, thereby controlling the activity of said transcription factor.

2. The cell of claim 1, wherein said reporter gene encodes a fluorescent or chemiluminescent protein, a protein capable of generating a chemiluminescent or fluorescent compound, or a protein capable of generating a spectroscopically detectable compound.

3. The cell of claim 1 or 2, wherein said response element is a tetracycline response element.

4. The cell of any one of claims 1 to 3, wherein said transcription factor comprises a Tet repressor or a Tet repressor mutant.

5. The cell of any one of claims 1 to 3, wherein said transcription factor comprises a reverse Tet repressor.

6. The cell of claim 4 or 5, wherein said transcription factor furthermore comprises a eukaryotic transcription activation domain.

7. The cell of any one of claims 4 to 6, wherein said transcription factor furthermore comprises a eukaryotic transcription repression domain.

8. The cell of any one of claims 1 to 7, wherein said peptide is fused to the N-terminus or the C-terminus of said polypeptide.

9. The cell of any one of claims 1 to 8, wherein said polypeptide of interest is selected from β-catenin, a Smad family protein, p53, MDM2, PARC, nucleophosmin, glucocorticoid receptor and viral proteins.

10. The cell of any one of claims 4 or 6 to 9, wherein said peptide is capable of binding to and regulating the DNA-binding activity of said Tet repressor or Tet repressor mutant.

11. The cell of any one of claims 5 to 9, wherein said peptide is capable of binding to and regulating the DNA-binding activity of said reverse Tet repressor.

12. The cell of claim 10, wherein
(a) said peptide comprises or consists of the sequence of any one of SEQ ID NOs. 1 to 34;
(b) said peptide is encoded by a first nucleic acid sequence which hybridizes with a second nucleic acid sequence, said second nucleic acid sequence being fully complementary to a third nucleic acid sequence encoding a peptide consisting of the sequence of any one of SEQ ID NOs: 1 to 34; or
(c) said peptide comprises or consists of an amino acid sequence which is at least 75% identical to the sequence of any one of SEQ ID NOs. 1 to 34.

13. The cell of claim 11, wherein
(a) said peptide comprises or consists of the sequence of SEQ ID NOs. 35 to 43;
(b) said peptide is encoded by a first nucleic acid sequence which hybridizes with a second nucleic acid sequence, said second nucleic acid sequence being fully complementary to a third nucleic acid sequence encoding a peptide consisting of the sequence of any one of SEQ ID NOs: 35 to 43; or
(c) said peptide comprises or consists of an amino acid sequence which is at least 75% identical to the sequence of any one of SEQ ID NOs. 35 to 43.

14. The cell of any one of the preceding claims, wherein said cell is a S. *cerevisiae* cell, an invertebrate cell or a vertebrate cell.

15. A method of determining the presence of a polypeptide of interest in the nucleus of a eukaryotic cell, said method comprising:
(a) culturing the cell as defined in any one of claims 1 to 14; and
(b) determining whether the reporter gene comprised in said cell is expressed.

16. A method of identifying a compound capable of altering the nuclear localization of a polypeptide of interest, said method comprising the method as defined in claim 15, wherein said culturing is effected in the presence and, independently, in the absence of a test compound, respectively, and wherein a difference in expression of said reporter gene in the presence of the test compound as compared to the absence of said test compound is indicative of the test compound being a compound capable of altering the nuclear localization of said polypeptide of interest.

17. The method of claim 15 or 16, wherein said cell is the cell as defined in any one of claims 2 to 14 and said determining is effected by determining the amount of fluorescence generated by said fluorescent protein or the amount of said chemiluminescent, fluorescent, or spectroscopically detectable compound.

18. Use of the cell of any one of claims 1 to 14 or of the third nucleic acid as defined in any one of claims 1 to 14 for determining presence of said polypeptide of interest in the nucleus.

19. Use of the cell of any one of claims 1 to 14 or of the third nucleic acid as defined in any one of claims 1 to 14 for identifying a compound capable of interfering with the nuclear localization of said polypeptide of interest..

20. Use of the third nucleic acid as defined in any one of claims 1 to 14 for transfecting, nucleofecting or transducing a eukaryotic cell.

21. A kit for transfecting, nucleofecting or transducing a eukaryotic cell and/or for performing the method as defined in any one of claims 15 to 17, said kit comprising first, second and third nucleic acids as defined in any one of claims 1 to 14.


SEQUENCE LISTING

<110>  Friedrich-Alexander-Universität Erlangen-Nürnberg

<120>  Means and methods for identifying compounds altering nuclear
       localization of proteins

<130>  S1330 EP

<160>  43

<170>  PatentIn version 3.3

<210>  1
<211>  12
<212>  PRT
<213>  artificial sequence

<220>
<223>  /note="Description of artificial sequence: transcription inducing
       peptide"

<400>  1

Trp Thr Trp Asn Ala Tyr Ala Phe Ala Ala Pro Ser
1               5                   10


<210>  2
<211>  12
<212>  PRT
<213>  artificial sequence

<220>
<223>  /note="Description of artificial sequence: transcription inducing
       peptide"

<220>
<221>  variation
<222>  1
<223>  /replace="Tyr"
       /replace="Thr"
       /replace="Leu"

<400>  2

Ser Thr Trp Asn Ala Tyr Ala Phe Ala Ala Pro Ser
1               5                   10


<210>  3
<211>  12
<212>  PRT
<213>  artificial sequence

<220>
<223>  /note="Description of artificial sequence: transcription inducing
       peptide"

<400>  3

Trp Ser Trp Asn Ala Tyr Ala Phe Ala Ala Pro Ser
1               5                   10


<210>  4
<211>  12
<212>  PRT

<213>   artificial sequence

<220>
<223>   /note="Description of artificial sequence: transcription inducing
        peptide"

<220>
<221>   variation
<222>   4
<223>   /replace="Leu"
        /replace="Phe"
        /replace="Glu"

<400>   4

Trp Thr Trp Met Ala Tyr Ala Phe Ala Ala Pro Ser
1               5                   10


<210>   5
<211>   12
<212>   PRT
<213>   artificial sequence

<220>
<223>   /note="Description of artificial sequence: transcription inducing
        peptide"

<220>
<221>   variation
<222>   5
<223>   /replace="Val"
        /replace="Ile"
        /replace="Pro"
        /replace="Thr"
        /replace="Tyr"
        /replace="Gly"

<400>   5

Trp Thr Trp Asn Ser Tyr Ala Phe Ala Ala Pro Ser
1               5                   10


<210>   6
<211>   12
<212>   PRT
<213>   artificial sequence

<220>
<223>   /note="Description of artificial sequence: transcription inducing
        peptide"

<220>
<221>   variation
<222>   6
<223>   /replace="Trp"
        /replace="Asn"
        /replace="Gly"
        /replace="Met"
        /replace="Ser"
        /replace="Lys"
        /replace="Cys"
        /replace="Ile"
        /replace="Phe"
        /replace="Gln"

<400>   6

Trp Thr Trp Asn Ala Thr Ala Phe Ala Ala Pro Ser
1               5                   10

<210>  7
<211>  12
<212>  PRT
<213>  artificial sequence

<220>
<223>  /note="Description of artificial sequence: transcription inducing
       peptide"

<220>
<221>  variation
<222>  7
<223>  /replace="Trp"
       /replace="Gly"
       /replace="Glu"
       /replace="Gln"

<400>  7

Trp Thr Trp Asn Ala Tyr Ser Phe Ala Ala Pro Ser
1               5                   10

<210>  8
<211>  12
<212>  PRT
<213>  artificial sequence

<220>
<223>  /note="Description of artificial sequence: transcription inducing
       peptide"

<220>
<221>  variation
<222>  8
<223>  /replace="Tyr"

<400>  8

Trp Thr Trp Asn Ala Tyr Ala Trp Ala Ala Pro Ser
1               5                   10

<210>  9
<211>  12
<212>  PRT
<213>  artificial sequence

<220>
<223>  /note="Description of artificial sequence: transcription inducing
       peptide"

<220>
<221>  variation
<222>  9
<223>  /replace="Val"
       /replace="Gly"
       /replace="Cys"
       /replace="Trp"
       /replace="Arg"

<400>  9

Trp Thr Trp Asn Ala Tyr Ala Phe Thr Ala Pro Ser
1               5                   10

<210>  10
<211>  12
<212>  PRT
<213>  artificial sequence

<220>
<223>  /note="Description of artificial sequence: transcription inducing
       peptide"

<220>
<221>  variation
<222>  10
<223>  /replace="Asp"
       /replace="Val"
       /replace="Leu"
       /replace="Glu"
       /replace="Tyr"

<400>  10

Trp Thr Trp Asn Ala Tyr Ala Phe Ala Met Pro Ser
1               5                   10

<210>  11
<211>  12
<212>  PRT
<213>  artificial sequence

<220>
<223>  /note="Description of artificial sequence: transcription inducing
       peptide"

<220>
<221>  variation
<222>  11
<223>  /replace="Ala"
       /replace="Ser"
       /replace="Gly"
       /replace="Trp"
       /replace="Leu"

<400>  11

Trp Thr Trp Asn Ala Tyr Ala Phe Ala Ala Tyr Ser
1               5                   10

<210>  12
<211>  12
<212>  PRT
<213>  artificial sequence

<220>
<223>  /note="Description of artificial sequence: transcription inducing
       peptide"

<220>
<221>  variation
<222>  12
<223>  /replace="Asn"
       /replace="Ala"
       /replace="Thr"
       /replace="Tyr"

/replace="Trp"
/replace="Gly"

<400>  12

Trp Thr Trp Asn Ala Tyr Ala Phe Ala Ala Pro Arg
1               5                   10


<210>  13
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<400>  13

Trp Trp Thr Trp Asn Ala Tyr Ala Phe Ala Ala Pro Ser
1               5                   10


<210>  14
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  2
<223>  /replace="Tyr"
       /replace="Thr"
       /replace="Leu"

<400>  14

Trp Ser Thr Trp Asn Ala Tyr Ala Phe Ala Ala Pro Ser
1               5                   10


<210>  15
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<400>  15

Trp Trp Ser Trp Asn Ala Tyr Ala Phe Ala Ala Pro Ser
1               5                   10


<210>  16
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  5
<223>  /replace="Leu"
       /replace="Phe"
       /replace="Glu"

<400>  16

Trp Trp Thr Trp Met Ala Tyr Ala Phe Ala Ala Pro Ser
1               5                   10


<210>  17
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  6
<223>  /replace="Val"
       /replace="Ile"
       /replace="Pro"
       /replace="Thr"
       /replace="Tyr"
       /replace="Gly"

<400>  17

Trp Trp Thr Trp Asn Ser Tyr Ala Phe Ala Ala Pro Ser
1               5                   10


<210>  18
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  7
<223>  /replace="Trp"
       /replace="Asn"
       /replace="Gly"
       /replace="Met"
       /replace="Ser"
       /replace="Lys"
       /replace="Cys"
       /replace="Ile"
       /replace="Phe"
       /replace="Gln"

<400>  18

Trp Trp Thr Trp Asn Ala Thr Ala Phe Ala Ala Pro Ser
1               5                   10


<210>  19
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  8
<223>  /replace="Trp"
       /replace="Gly"
       /replace="Glu"
       /replace="Gln"

<400>  19

Trp Trp Thr Trp Asn Ala Tyr Ser Phe Ala Ala Pro Ser
1               5                   10

<210>  20
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  9
<223>  /replace="Tyr"

<400>  20

Trp Trp Thr Trp Asn Ala Tyr Ala Trp Ala Ala Pro Ser
1               5                   10

<210>  21
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  10
<223>  /replace="Val"
       /replace="Gly"
       /replace="Cys"
       /replace="Trp"
       /replace="Arg"

<400>  21

Trp Trp Thr Trp Asn Ala Tyr Ala Phe Thr Ala Pro Ser
1               5                   10

<210>  22
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  11
<223>  /replace="Asp"
       /replace="Val"
       /replace="Leu"
       /replace="Glu"
       /replace="Tyr"

<400>  22

Trp Trp Thr Trp Asn Ala Tyr Ala Phe Ala Met Pro Ser
1               5                   10


<210>  23
<211>  13
<212>  PRT
<213>  artificial sequence

<220>
<223>  /note="Description of artificial sequence: transcription inducing
       peptide"

<220>
<221>  variation
<222>  1
<223>  /replace="Phe"
       /replace="His"
       /replace="Tyr"

<220>
<221>  variation
<222>  12
<223>  /replace="Ala"
       /replace="Ser"
       /replace="Gly"
       /replace="Trp"
       /replace="Leu"

<400>  23

Trp Trp Thr Trp Asn Ala Tyr Ala Phe Ala Ala Tyr Ser
1               5                   10


<210>  24
<211>  13
<212>  PRT
<213>  artificial sequence

<220>
<223>  /note="Description of artificial sequence: transcription inducing
       peptide"

<220>
<221>  variation

<222>  1
<223>  /replace="Phe"
       /replace="His"
       /replace="Tyr"

<220>
<221>  variation
<222>  13
<223>  /replace="Asn"
       /replace="Ala"
       /replace="Thr"
       /replace="Tyr"
       /replace="Trp"
       /replace="Gly"

<400>  24

Trp Trp Thr Trp Asn Ala Tyr Ala Phe Ala Ala Pro Arg
1               5                   10


<210>  25
<211>  16
<212>  PRT
<213>  artificial sequence

<220>
<223>  /note="Description of artificial sequence: transcription inducing
       peptide"

<400>  25

Asp Asp Ser Val Leu Ala Ala Arg Ala Arg Met Trp Met Trp His Trp
1               5                   10                  15


<210>  26
<211>  16
<212>  PRT
<213>  artificial sequence

<220>
<223>  /note="Description of artificial sequence: transcription inducing
       peptide"

<220>
<221>  variation
<222>  1
<223>  /replace="Cys"
       /replace="Glu"
       /replace="Phe"
       /replace="Gly"
       /replace="His"
       /replace="Ile"
       /replace="Lys"
       /replace="Leu"
       /replace="Met"
       /replace="Asn"
       /replace="Pro"
       /replace="Gln"
       /replace="Arg"
       /replace="Ser"
       /replace="Thr"
       /replace="Val"
       /replace="Trp"
       /replace="Tyr"

<400>  26

Ala Asp Ser Val Leu Ala Ala Arg Ala Arg Met Trp Met Trp His Trp
1               5                   10                  15

<210>  27
<211>  16
<212>  PRT
<213>  artificial sequence

<220>
<223>  /note="Description of artificial sequence: transcription inducing
       peptide"

<220>
<221>  variation
<222>  2
<223>  /replace="Cys"
       /replace="Glu"
       /replace="Phe"
       /replace="Gly"
       /replace="His"
       /replace="Ile"
       /replace="Lys"
       /replace="Leu"
       /replace="Met"
       /replace="Asn"
       /replace="Pro"
       /replace="Gln"
       /replace="Arg"
       /replace="Ser"
       /replace="Thr"
       /replace="Val"
       /replace="Trp"
       /replace="Tyr"

<400>  27

Asp Ala Ser Val Leu Ala Ala Arg Ala Arg Met Trp Met Trp His Trp
1               5                   10                  15

<210>  28
<211>  16
<212>  PRT
<213>  artificial sequence

<220>
<223>  /note="Description of artificial sequence: transcription inducing
       peptide"

<220>
<221>  variation
<222>  3
<223>  /replace="Asp"
       /replace="Cys"
       /replace="Glu"
       /replace="Phe"
       /replace="Gly"
       /replace="His"
       /replace="Ile"
       /replace="Lys"
       /replace="Leu"
       /replace="Met"
       /replace="Asn"
       /replace="Pro"
       /replace="Gln"
       /replace="Arg"

/replace="Thr"
/replace="Val"
/replace="Trp"
/replace="Tyr"

<400> 28

Asp Asp Ala Val Leu Ala Ala Arg Ala Arg Met Trp Met Trp His Trp
1               5                   10                  15


<210> 29
<211> 16
<212> PRT
<213> artificial sequence

<220>
<223> /note="Description of artificial sequence: transcription inducing
      peptide"

<220>
<221> variation
<222> 4
<223> /replace="Asp"
      /replace="Cys"
      /replace="Glu"
      /replace="Phe"
      /replace="Gly"
      /replace="His"
      /replace="Ile"
      /replace="Lys"
      /replace="Leu"
      /replace="Met"
      /replace="Asn"
      /replace="Pro"
      /replace="Gln"
      /replace="Arg"
      /replace="Ser"
      /replace="Thr"
      /replace="Trp"
      /replace="Tyr"

<400> 29

Asp Asp Ser Ala Leu Ala Ala Arg Ala Arg Met Trp Met Trp His Trp
1               5                   10                  15


<210> 30
<211> 16
<212> PRT
<213> artificial sequence

<220>
<223> /note="Description of artificial sequence: transcription inducing
      peptide"

<220>
<221> variation
<222> 5
<223> /replace="Cys"
      /replace="Asp"
      /replace="Glu"
      /replace="Phe"
      /replace="Gly"
      /replace="His"
      /replace="Ile"
      /replace="Lys"

/replace="Met"
/replace="Asn"
/replace="Pro"
/replace="Gln"
/replace="Arg"
/replace="Ser"
/replace="Thr"
/replace="Val"
/replace="Trp"
/replace="Tyr"

<400> 30

Asp Asp Ser Val Ala Ala Ala Arg Ala Arg Met Trp Met Trp His Trp
1               5                   10                  15


<210> 31
<211> 16
<212> PRT
<213> artificial sequence

<220>
<223> /note="Description of artificial sequence: transcription inducing
peptide"

<220>
<221> variation
<222> 8
<223> /replace="Cys"
/replace="Asp"
/replace="Glu"
/replace="Phe"
/replace="Gly"
/replace="His"
/replace="Ile"
/replace="Lys"
/replace="Leu"
/replace="Met"
/replace="Asn"
/replace="Pro"
/replace="Gln"
/replace="Ser"
/replace="Thr"
/replace="Val"
/replace="Trp"
/replace="Tyr"

<400> 31

Asp Asp Ser Val Leu Ala Ala Ala Ala Arg Met Trp Met Trp His Trp
1               5                   10                  15


<210> 32
<211> 16
<212> PRT
<213> artificial sequence

<220>
<223> /note="Description of artificial sequence: transcription inducing
peptide"

<220>
<221> variation
<222> 9
<223> /replace="Cys"
/replace="Asp"

/replace="Glu"
/replace="Phe"
/replace="Gly"
/replace="His"
/replace="Ile"
/replace="Lys"
/replace="Met"
/replace="Asn"
/replace="Pro"
/replace="Gln"
/replace="Arg"
/replace="Ser"
/replace="Thr"
/replace="Val"
/replace="Trp"
/replace="Tyr"

<400> 32

Asp Asp Ser Val Leu Ala Ala Arg Leu Arg Met Trp Met Trp His Trp
1               5                   10                  15


<210> 33
<211> 16
<212> PRT
<213> artificial sequence

<220>
<223> /note="Description of artificial sequence: transcription inducing
peptide"

<220>
<221> variation
<222> 10
<223> /replace="Cys"
/replace="Asp"
/replace="Glu"
/replace="Phe"
/replace="Gly"
/replace="His"
/replace="Ile"
/replace="Lys"
/replace="Leu"
/replace="Met"
/replace="Asn"
/replace="Pro"
/replace="Gln"
/replace="Ser"
/replace="Thr"
/replace="Val"
/replace="Trp"
/replace="Tyr"

<400> 33

Asp Asp Ser Val Leu Ala Ala Arg Ala Ala Met Trp Met Trp His Trp
1               5                   10                  15


<210> 34
<211> 16
<212> PRT
<213> artificial sequence

<220>
<223> /note="Description of artificial sequence: transcription inducing
peptide"

<220>
<221>  variation
<222>  12
<223>  /replace="Cys"
       /replace="Asp"
       /replace="Glu"
       /replace="Phe"
       /replace="Gly"
       /replace="His"
       /replace="Ile"
       /replace="Lys"
       /replace="Leu"
       /replace="Met"
       /replace="Asn"
       /replace="Pro"
       /replace="Gln"
       /replace="Arg"
       /replace="Ser"
       /replace="Thr"
       /replace="Val"
       /replace="Tyr"

<400>  34

Asp Asp Ser Val Leu Ala Ala Arg Ala Arg Met Ala Met Trp His Trp
1               5                   10                  15


<210>  35
<211>  16
<212>  PRT
<213>  artificial sequence

<220>
<223>  /note="Description of artificial sequence: transcription inducing
       peptide"

<400>  35

Thr Gly Ala Ile Ile Met Ala Val Ala Arg Leu Leu Pro Ser Trp Asn
1               5                   10                  15


<210>  36
<211>  16
<212>  PRT
<213>  artificial sequence

<220>
<223>  /note="Description of artificial sequence: transcription inducing
       peptide"

<220>
<221>  variation
<222>  1
<223>  /replace="Cys"
       /replace="Asp"
       /replace="Glu"
       /replace="Phe"
       /replace="Gly"
       /replace="His"
       /replace="Ile"
       /replace="Lys"
       /replace="Leu"
       /replace="Met"
       /replace="Asn"
       /replace="Pro"

/replace="Gln"
/replace="Arg"
/replace="Ser"
/replace="Val"
/replace="Trp"
/replace="Tyr"

<400> 36

Ala Gly Ala Ile Ile Met Ala Val Ala Arg Leu Leu Pro Ser Trp Asn
1               5                   10                  15


<210> 37
<211> 16
<212> PRT
<213> artificial sequence

<220>
<223> /note="Description of artificial sequence: transcription inducing
peptide"

<220>
<221> variation
<222> 2
<223> /replace="Cys"
/replace="Asp"
/replace="Glu"
/replace="Phe"
/replace="His"
/replace="Ile"
/replace="Lys"
/replace="Leu"
/replace="Met"
/replace="Asn"
/replace="Pro"
/replace="Gln"
/replace="Arg"
/replace="Ser"
/replace="Thr"
/replace="Val"
/replace="Trp"
/replace="Tyr"

<400> 37

Thr Ala Ala Ile Ile Met Ala Val Ala Arg Leu Leu Pro Ser Trp Asn
1               5                   10                  15


<210> 38
<211> 16
<212> PRT
<213> artificial sequence

<220>
<223> /note="Description of artificial sequence: transcription inducing
peptide"

<220>
<221> variation
<222> 3
<223> /replace="Cys"
/replace="Asp"
/replace="Glu"
/replace="Phe"
/replace="Gly"
/replace="His"

/replace="Ile"
/replace="Leu"
/replace="Met"
/replace="Asn"
/replace="Pro"
/replace="Gln"
/replace="Arg"
/replace="Ser"
/replace="Thr"
/replace="Val"
/replace="Trp"
/replace="Tyr"

<400> 38

Thr Gly Lys Ile Ile Met Ala Val Ala Arg Leu Leu Pro Ser Trp Asn
1               5                   10                  15


<210> 39
<211> 16
<212> PRT
<213> artificial sequence

<220>
<223> /note="Description of artificial sequence: transcription inducing
      peptide"

<220>
<221> variation
<222> 5
<223> /replace="Cys"
      /replace="Asp"
      /replace="Glu"
      /replace="Phe"
      /replace="Gly"
      /replace="His"
      /replace="Lys"
      /replace="Leu"
      /replace="Met"
      /replace="Asn"
      /replace="Pro"
      /replace="Gln"
      /replace="Arg"
      /replace="Ser"
      /replace="Thr"
      /replace="Val"
      /replace="Trp"
      /replace="Tyr"

<400> 39

Thr Gly Ala Ile Ala Met Ala Val Ala Arg Leu Leu Pro Ser Trp Asn
1               5                   10                  15


<210> 40
<211> 16
<212> PRT
<213> artificial sequence

<220>
<223> /note="Description of artificial sequence: transcription inducing
      peptide"

<220>
<221> variation
<222> 6

<223>  /replace="Cys"
      /replace="Asp"
      /replace="Glu"
      /replace="Phe"
      /replace="Gly"
      /replace="His"
      /replace="Ile"
      /replace="Lys"
      /replace="Leu"
      /replace="Asn"
      /replace="Pro"
      /replace="Gln"
      /replace="Arg"
      /replace="Ser"
      /replace="Thr"
      /replace="Val"
      /replace="Trp"
      /replace="Tyr"

<400>  40

Thr Gly Ala Ile Ile Ala Ala Val Ala Arg Leu Leu Pro Ser Trp Asn
1               5                   10                  15


<210>  41
<211>  16
<212>  PRT
<213>  artificial sequence

<220>
<223>  /note="Description of artificial sequence: transcription inducing
       peptide"

<220>
<221>  variation
<222>  12
<223>  /replace="Cys"
       /replace="Asp"
       /replace="Glu"
       /replace="Phe"
       /replace="Gly"
       /replace="His"
       /replace="Ile"
       /replace="Lys"
       /replace="Met"
       /replace="Asn"
       /replace="Pro"
       /replace="Gln"
       /replace="Arg"
       /replace="Ser"
       /replace="Thr"
       /replace="Val"
       /replace="Trp"
       /replace="Tyr"

<400>  41

Thr Gly Ala Ile Ile Met Ala Val Ala Arg Leu Ala Pro Ser Trp Asn
1               5                   10                  15


<210>  42
<211>  16
<212>  PRT
<213>  artificial sequence

<220>

<223>   /note="Description of artificial sequence: transcription inducing
        peptide"

<220>
<221>   variation
<222>   14
<223>   /replace="Cys"
        /replace="Asp"
        /replace="Glu"
        /replace="Phe"
        /replace="Gly"
        /replace="His"
        /replace="Ile"
        /replace="Lys"
        /replace="Leu"
        /replace="Met"
        /replace="Asn"
        /replace="Pro"
        /replace="Gln"
        /replace="Arg"
        /replace="Thr"
        /replace="Val"
        /replace="Trp"
        /replace="Tyr"

<400>   42

Thr Gly Ala Ile Ile Met Ala Val Ala Arg Leu Leu Pro Ala Trp Asn
1               5                   10                  15


<210>   43
<211>   16
<212>   PRT
<213>   artificial sequence

<220>
<223>   /note="Description of artificial sequence: transcription inducing
        peptide"

<220>
<221>   variation
<222>   16
<223>   /replace="Cys"
        /replace="Asp"
        /replace="Glu"
        /replace="Phe"
        /replace="Gly"
        /replace="His"
        /replace="Ile"
        /replace="Lys"
        /replace="Leu"
        /replace="Met"
        /replace="Pro"
        /replace="Gln"
        /replace="Arg"
        /replace="Ser"
        /replace="Thr"
        /replace="Val"
        /replace="Trp"
        /replace="Tyr"

<400>   43

Thr Gly Ala Ile Ile Met Ala Val Ala Arg Leu Leu Pro Ser Trp Ala
1               5                   10                  15